# EUROPEAN PATENT APPLICATION

(11) **EP 4 238 759 A1**
(43) Date of publication of application: **06.09.2023**
(21) Application number: 21886208.4
(22) Date of filing: 26.10.2021
(51) Int. Cl.: B32B 27/00, C08F 20/00, C09J 11/04, C09J 11/08, C09J 131/00, C09J 133/06, C09J 201/00, A61F 13/02, C09J 7/22, C09J 7/24, C09J 7/38

(54) **RESIN FOR ADHESIVE SHEET, AND ADHESIVE SHEET**

(30) Priority: 29.10.2020 JP 2020181692; 28.04.2021 JP 2021075785
(71) Applicant: Nitto Denko Corporation, Ibaraki-shi, Osaka 567-8680 (JP)
(72) Inventor: HIRANO, Keisuke, Ibaraki-shi, Osaka 567-8680 (JP)
(74) Representative: Gill Jennings & Every LLP
(86) International application number: PCT/JP2021/039506
(87) International publication number: WO 2022/092090

(57) **Abstract**

Provided is a resin for PSA sheet that has adhesive reliability such that it does not peel off or shift out of place even when applied to a flexible adherend, an adherend with brittle surface or an adherend with low strength; and that can be peeled off without adherend damage during removal. The PSA sheet resin provided has a storage modulus at 25 °C in the range of 10 MPa to 500 MPa, a storage modulus at 37 °C in the range of 0.5 MPa to 20 MPa, and a surface hardness at 37 °C in the range of 0.1 MPa to 2 MPa.

## Description

### [Technical Field]

The present invention relates to a resin for pressure-sensitive adhesive sheets and a pressure-sensitive adhesive sheet.

The present application claims priority based on Japanese Patent Application No. 2020-181692 filed on October 29, 2020 and Japanese Patent Application No. 2021-075785 filed on April 28, 2021; and the entire contents thereof are incorporated herein by reference.

### [Background Art]

In general, pressure-sensitive adhesive (PSA) exists as a soft solid (a viscoelastic material) in a room temperature range and has a property to adhere easily to an adherend with some pressure applied. For such a property, PSA has been widely used in various fields as a supported PSA sheet having a PSA layer on a support or as a support-less PSA sheet free of a support. Technical documents about PSA sheets include Patent Documents 1 to 4.

### [Citation List]

### [Patent Literature]

[Patent Document 1] Japanese Patent No. 6591841
[Patent Document 2] Japanese Patent Application Publication No. 2017-39856
[Patent Document 3] Japanese Patent Application Publication No. 2020-6166
[Patent Document 4] Japanese Patent Application Publication No. 2019-14852

### [Summary of Invention]

### [Technical Problem]

Depending on how they are used, PSA sheets can be required to have properties such as post-application peel resistance, displacement resistance, and removability without leftover adhesive residue (clean removal). For instance, on flexible adherends such as paper and human skin, peeling often occurs due to adherend surface deformation, etc. Thus, it is not easy to achieve properties such as peel resistance and displacement resistance for long terms. If a strong adhesive PSA is used to form firm bonding to the adherend and prevent peeling, problems may occur such as leaving adhesive residue upon removal of the PSA sheet from the adherend because of the strong adhesiveness, or leaving a piece of the PSA sheet on the adherend due to partial breakage of the PSA sheet (e.g., nonwoven fabric substrate, etc.). Such a PSA sheet undesirably requires a lot of effort to remove. In the case of these flexible adherends, adherends with brittle surfaces, or adherends with low mechanical strength, removal from the adherend tends to be even more difficult; and the adherend can be damaged or even destroyed. In particular, PSA sheets applied to human skin are required to not cause skin rashes while being applied as well as to have removability with little irritation or damage to the skin during removal. In such applications, it is important that the PSA can be removed from the skin with little or no pain. However, for instance, if the PSA is hardened for such easy removal, the adhesive strength will decrease, likely causing peeling or displacement when it is applied and fixed, and adhesive reliability cannot be obtained.

About these problems, Patent Document 1 suggests a method to reduce adhesive residue by modifying a urethane-based PSA with a silicone-based compound. No studies have been made about combining it with properties to resist peeling and displacement. According to Patent Document 2, a component having a UV-reactive functional group is introduced into a PSA and reaction caused by post-application UV irradiation brings about removability, preventing leftover adhesive residue. However, it is not easy to handle as it requires post-application UV irradiation. The PSA disclosed in Patent Document 2 also has low adhesive strength and thus is not suitable for applications requiring properties to resist peeling and displacement. Patent Document 3 suggests adding a specific fatty acid ester to the PSA to reduce irritation to the skin; however, with the weak adhesiveness, properties such as peel resistance and displacement resistance cannot be expected, either. On the other hand, Patent Document 4 teaches a PSA having relatively high adhesive strength and suggests wash removal with organic solvent when adhesive residue is left. However, the organic solvent needs to be wiped off when the PSA sheet is removed from the adherend. This limits convenience, places of use, etc.

The present invention has been made in view of the above circumstances with an objective to provide a PSA sheet that has adhesive reliability such that it does not peel off or shift out of place even when applied to a flexible adherend, an adherend with brittle surface or an adherend with low strength, with the PSA sheet being removable without damaging to the adherend during removal. Another related objective is to provide a PSA sheet resin for use in such a PSA sheet.

### [Solution to Problem]

This description provides a resin for PSA sheets (a PSA sheet resin). The resin has a storage modulus at 25 °C in the range of 10 MPa to 500 MPa, a storage modulus at 37 °C in the range of 0.5 MPa to 20 MPa, and a surface hardness at 37 °C in the range of 0.1 MPa to 2 MPa. By using a resin satisfying these properties, it is possible to prepare a PSA sheet that does not easily peel off or shift out of place, yet can be peeled off for removal without damage to the adherend. In particular, having these properties, the resin (e.g., as a sheet) placed in the PSA sheet deforms with gentle stress relaxation to conform to the adherend; and therefore, it helps bring about peel resistance and displacement resistance in the PSA sheet. On the other hand, the resin suitably resists deformation and reduces the local concentration of stress on the adherend. Thus, it can prevent damage to the adherend caused by the stress during such removal whereby the PSA sheet can be peeled off

In some preferable embodiments, the resin has an unloading curve displacement in the range of 400 nm to 1500 nm in nanoindentation carried out at a temperature of 37 °C, an indentation depth of 1000 nm, and indentation/removal rates of 1000 nm/s. The PSA sheet prepared using a resin satisfying this property preferably bring about the effect of the art disclosed herein.

In some preferable embodiments, the resin has an area inside a load-displacement curve of 5 pJ or greater when the load is 0 or less in nanoindentation carried out at a temperature of 37 °C, an indentation depth of 1000 nm, and indentation/removal rates of 1000 nm/s. The PSA sheet prepared using a resin satisfying this property preferably bring about the effect of the art disclosed herein.

In some embodiments, the resin has a glass transition temperature (Tg) in the range of 5 °C to 40 °C. With the use of the resin having a Tg in this range, flexibility increases at a temperature near, at or above the Tg; and it tends to gently conform to the adherend.

In some preferable embodiments, the resin is an acrylic resin. The use of acrylic resin as the resin can preferably bring about a structure having a specific storage modulus and surface hardness.

This description provides a PSA sheet having a layer A formed from a resin disclosed herein. According to the PSA sheet in this embodiment, the layer A in the PSA sheet gently conforms to the adherend whereby peel resistance and displacement resistance can be obtained. When the PSA sheet is removed from the adherend, the layer-A-containing PSA sheet suitably resists deformation during removal and reduces the local concentration of stress on the adherend. Thus, removal can be completed while preventing damage to the adherend caused by the stress.

The PSA sheet according to some preferable embodiments has an adhesive face (bonding surface) having a 180° peel strength on stainless steel plate (on-SUS adhesive strength) of 4 N/10mm or greater. The PSA sheet having such an adhesive face can provide good adhesive fixation to the adherend. Even when the adhesive surface has at least the prescribed adhesive strength as described above, because of the layer A, the PSA sheet disclosed herein can be peeled off the adherend without leftover adhesive residue.

The PSA sheet provided by this description may have a layer A that has a storage modulus at 25 °C in the range of 10 MPa to 500 MPa and a storage modulus at 37 °C in the range of 0.5 MPa to 20 MPa. The PSA sheet has an adhesive face having a 180° peel strength on stainless steel plate of 4 N/10mm or greater. The PSA sheet in this embodiment can provide good adhesive fixation to the adherend and the layer A in the PSA sheet gently conforms to the adherend whereby peel resistance and displacement resistance can be obtained. When the PSA sheet is removed from the adherend, the layer-A-containing PSA sheet suitably resists deformation during removal and reduces the local concentration of stress on the adherend. Thus, removal can be completed while preventing the occurrence of stress-induced adherend damage.

In addition to the layer A, the PSA sheet according to some preferable embodiments has a layer B constituting the adhesive face. With the layer B (typically a PSA layer) constituting the adhesive surface, while maintaining the layer A's function (bulk stress control), the layer B is designed to enable control (interfacial control) of tightness of adhesion to the adherend. While no particular limitations are imposed, as the layer B, an acrylic PSA layer is preferably used. The layer B formed of acrylic PSA can preferably bring about at least a certain adhesive strength (on-SUS adhesive strength).

The layer B may have a thickness of 0.5 µm or greater and 100 µm or less. In some embodiments, the layer B thickness can be 0.5 µm or greater and less than 10 µm. In an embodiment having such a thin layer B, the effect of the art disclosed herein can be preferably obtained.

In addition to the layer A, the PSA sheet according to some embodiments has a layer C. Here, the layer C is placed on the opposite side of the layer A's adhesive face. When the adhesive face, layer A and layer C are placed in this order, the layer C's function can be added to the PSA sheet while effectively providing the layer A's function (bulk stress control) to the adhesive face. For instance, when the layer C is a PSA layer, not only the adhesive face (the first adhesive face), but also the layer C side of the PSA sheet serves as an adhesive face (the second adhesive face), making the sheet adhesively double-faced for fixing function. In addition, for instance, when the layer C is provided with cushioning properties, the layer C can absorb layer C-side contours (e.g., the adherend shape) and external force to prevent their influence on the first adhesive face.

In some preferable embodiments, the layer C includes hollow particles, or has bubbles (cells, pores), or has both. By using a layer C having hollow particles and/or bubbles, the amount of energy required to remove the PSA sheet from the adherend increases whereby it may show greater resistance to peeling and displacement in addition to the effect of the layer A's presence. Such layer C effect does not impair the removability (adherend damage prevention) according to the layer A's presence, yet it helps achieve a higher level of the effect of the art disclosed herein (resisting to displacement and peeling off from the adherend while not damaging the adherend during removal). Because the layer C has cushioning properties, for instance, when a hard member is placed on the layer C side, the layer C can absorb the member's influence on the PSA sheet, allowing the adhesive face located on the opposite side to be unaffected. In some embodiments, the layer C is an acrylic PSA layer. The art disclosed herein is preferably implemented in an embodiment (typically as a double-faced PSA sheet) having an acrylic PSA layer as the layer C.

The PSA sheet disclosed herein is preferably used in an embodiment where the adhesive face is applied to human skin. The PSA sheet applied to human skin gently conforms to the application area according to the body temperature whereby it can achieve peel resistance and displacement resistance. For instance, it can also have resistance to peeling and displacement due to human movement. On the other hand, for removal of the PSA sheet, because of the layer A's presence, the PSA sheet suitably resists deformation during removal and reduces the local concentration of stress on the adherend. In particular, when compared to the adhesive strength and peel resistance, the PSA sheet disclosed herein has a low maximum stress value at the initial stage of removal. Thus, When the PSA is removed from the skin, there is little pulling (deformation) of the skin, allowing gentle peeling with little or no pain.

The PSA sheet disclosed herein is particularly suitable for fixing a sensor to human skin. The PSA sheet disclosed herein can be used in an application on human skin as described above. Even when a sensor is fixed via the PSA sheet to a specific site of a person, it does not easily peel off or shift out of place; and therefore, it is possible to exhibit the sensing function associated with the sensor-fixed site with high accuracy. The PSA sheet having such an ability to fix sensors to human body sites can be beneficial in fields such as medicine, healthcare and sport science.

This description can also provide a PSA sheet having a novel constitution suitable for bringing about the effect of the art disclosed herein. One constitution of such a PSA sheet is a PSA sheet laminated with a layer B (PSA layer) constituting an adhesive face. The PSA sheet has an adhesive face having a 180° peel strength on stainless steel plate of 4 N/10mm or greater. As the layer B, an acrylic PSA layer is preferably used. While no particular limitations are imposed, the layer B thickness can be 0.5 µm or greater and less than 10 µm.

### [Brief Description of Drawings]

Fig. 1 shows a cross-sectional diagram schematically illustrating the PSA sheet resin according to an embodiment.
Fig. 2 shows a cross-sectional diagram schematically illustrating the PSA sheet according to an embodiment.
Fig. 3 shows a cross-sectional diagram schematically illustrating the PSA sheet according to another embodiment.
Fig. 4 shows a cross-sectional diagram schematically illustrating a constitutional example of the PSA sheet with sensor.
Fig. 5 shows the peel stress distribution diagram of Example 1.
Fig. 6 shows the peel stress distribution diagram of Example 2.
Fig. 7 shows the peel stress distribution diagram of Comparative Example 1.

### [Description of Embodiments]

Preferable embodiments of the present invention are described below. Matters necessary to practice this invention other than those specifically referred to in this Description can be understood by a person skilled in the art based on the disclosure about implementing the invention in this Description and common technical knowledge at the time the application was filed. The present invention can be practiced based on the contents disclosed in this Description and common technical knowledge in the subject field. In the following drawings, components or units having the same functions may be described with the same symbols allocated and redundant description may be omitted or simplified. The embodiments illustrated in the drawings are schematic in order to clearly describe the present invention and the drawings do not accurately represent the size or scale of products actually provided.

As used herein, the term "PSA" refers to, as described earlier, a material that exists as soft solid (a viscoelastic material) in a room temperature range and has a property to adhere easily to an adherend with some pressure applied. As defined in *"*Adhesion: Fundamentals and Practice" by C. A. Dahlquist, McLaren & Sons (1966), P. 143), the PSA referred to herein can be a material that has a property satisfying complex tensile modulus E* (1Hz) < 10⁷ dyne/cm² (typically, a material that exhibits the described characteristics at 25 °C).

### <PSA sheet resin>

### (Constitutional example)

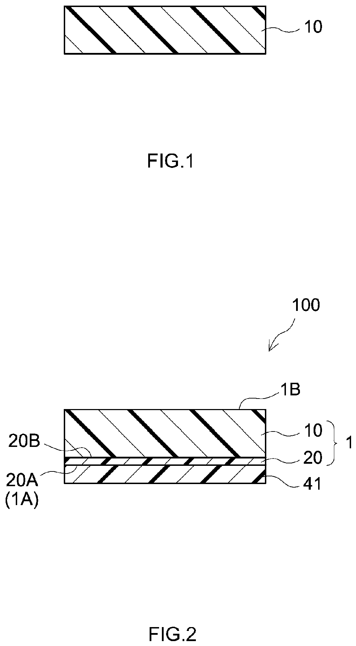

The form of the PSA sheet resin disclosed herein is not particularly limited. It may have, for instance, a cross-sectional structure schematically illustrated in Fig. 1. Resin sheet 10 shown in Fig. 1 is formed of a resin layer having a monolayer structure. Prior to use, resin sheet 10 may be in the form of a release-linered resin sheet with at least one face protected with a release liner.

### (Storage modulus at 25 °C (25°C storage modulus))

The PSA sheet resin disclosed herein has a storage modulus in the range of 10 MPa to 500 MPa at 25 °C. The 25°C storage modulus in this numerical range helps obtain suitable shape retention and stress relaxation; and when used as a PSA sheet material, peel resistance and displacement resistance to adherend are likely to be combined with prevention of damage to the adherend during removal. The 25°C storage modulus can be, for instance, about 30 MPa or higher, about 60 MPa or higher, or even about 90 MPa or higher. The 25°C storage modulus can be about 400 MPa or lower, about 300 MPa or lower, about 150 MPa or lower, or even about 100 MPa or lower.

### (Storage modulus at 37 °C (37°C storage modulus))

The PSA sheet resin disclosed herein has a storage modulus at 37°C in the range of 0.5 MPa to 20 MPa. The 37°C storage modulus in this numerical range helps obtain suitable shape retention and stress relaxation; and when used as a PSA sheet material, peel resistance and displacement resistance to adherend are likely to be combined with prevention of damage to the adherend during removal. For instance, in an application on human skin, the PSA sheet having a resin that satisfies this property gently conforms to the flexible application area according to the body temperature whereby it can obtain peel resistance and displacement resistance. It can also be resistant to peeling and displacement due to human movement. The 37°C storage modulus can be, for instance, about 0.6 MPa or higher, about 0.7 MPa or higher, or even about 0.8 MPa or higher (e.g., 1.0 MPa or higher). The 37°C storage modulus can be about 15 MPa or lower, about 10 MPa or lower, about 5 MPa or lower, or even about 3 MPa or lower (e.g., 1.5 MPa or lower).

### (Glass transition temperature)

While no particular limitations are imposed, the resin preferably has a glass transition temperature (Tg) in the range of 5 °C to 40 °C. With the use of the resin having a Tg in this range, flexibility increases at a temperature near, at or above the Tg; and it tends to gently conform to the adherend. The Tg is preferably 15 °C or higher, more preferably 20 °C or higher, possibly 25 °C or higher, or even 30 °C or higher. The Tg is preferably 35 °C or lower, possibly 30 °C or lower, or even 25 °C or lower. Resins with such Tg values become more flexible near human body temperature with increased stress relaxation properties. Thus, when used in a PSA sheet that is directly or indirectly applied to human skin, good conformability to adherend can be obtained.

The resin's viscoelastic properties (25°C and 37°C storage moduli, Tg) can be determined by dynamic viscoelastic analysis. In particular, they are determined by the methods described later in Examples. Based on the content of this description, the resin's viscoelastic properties can be adjusted through selection of resin components and composition, resin preparation conditions, etc. It is noted that, as used herein, the resin's Tg refers to the glass transition temperature determined from the tan δ peak temperature in dynamic viscoelastic analysis. The same applies to the Tg of a PSA as the layer B described later.

### (Surface hardness at 37 °C)

The resin disclosed herein has a surface hardness at 37 °C in the range of0. 1 MPa to 2 MPa. With the surface hardness in this numerical range, when placed in the PSA sheet, the resin undergoes gentle stress relaxation and deformation, conforming to the adherend; and therefore, it helps bring about peel resistance and displacement resistance. For instance, when using the resin as a PSA sheet material that is directly or indirectly applied to human skin, the resin has such surface hardness near human body temperature; and therefore, it can well conform to the adherend, preferably showing excellent properties. On the other hand, during removal of the PSA sheet, the resin suitably resists deformation and reduces the local concentration of stress on the adherend. Thus, it can help prevent damage to the adherend caused by the stress during such removal. The surface hardness is preferably 0.2 MPa or higher, more preferably 0.3 MPa or higher, or possibly 0.4 MPa or higher. The surface hardness is preferably 1.2 MPa or lower, more preferably 1.0 MPa or lower, yet more preferably 0.8 MPa or lower, or possibly even 0.6 MPa or lower (e.g., 0.5 MPa or lower).

### (Unloading curve displacement)

In some preferable embodiments, the resin has an unloading curve displacement in the range of 400 nm to 1500 nm in nanoindentation carried out at 37 °C. When placed in the PSA sheet, the resin having an unloading curve displacement of at least 400 nm has adequate viscosity and suitably conforms to the adherend, contributing to achieve peel resistance and displacement resistance. For instance, when using the resin as a PSA sheet material applied directly or indirectly to human skin, the resin satisfies the unloading curve displacement near the human body temperature; and therefore, it can preferably exhibit excellent properties. The unloading curve displacement is in the aforementioned range can lead to inhibition of deformation due to the load applied during the PSA sheet removal and suitable reduction of the stress on the adherend, contributing to prevent adherend damage caused by the stress during removal. The unloading curve displacement can be 500 nm or greater, 700 nm or greater, 900 nm or greater, or even 1100 nm or greater. The unloading curve displacement can be 1300 nm or less, 1000 nm or less, 800 nm or less, or even 600 nm or less.

### (Area inside load-displacement curve at load ≤ 0)

In some preferable embodiments, the resin has an area inside the load-displacement curve(s) of 5 pJ or greater when the load is 0 or less in nanoindentation carried out at 37 °C. When placed in the PSA sheet, the resin satisfying this property has adequate viscosity and suitably conforms to the adherend, contributing to achieve peel resistance and displacement resistance. For instance, when using the resin as a PSA sheet material applied directly or indirectly to human skin, the resin satisfies this property near the human body temperature; and therefore, it can preferably exhibit excellent properties. The inside-load-displacement-curve area (pJ) at load ≤ 0 can be 8 or greater, 10 or greater, or even 12 or greater. The maximum inside-load-displacement-curve area (pJ) at load ≤ 0 is not particularly limited. It can be, for instance, 50 or less, 30 or less, or even 15 or less.

The 37°C surface hardness, the unloading curve displacement and the inside-load-displacement-curve area at load ≤ 0 can be determined by nanoindentation carried out at a temperature of 37 °C, an indentation depth of 1000 nm, and indentation/removal rates (indentation rate and removal rate) of 1000 nm/s. In particular, they are measured by the methods described later in Examples. The resin's nanoindentation properties (surface hardness at 37 °C, unloading curve displacement and inside-load-displacement-curve area at load ≤ 0) can be adjusted based on this description through selection of resin components and composition, resin preparation conditions, etc.

### (Resin material)

The resin disclosed herein may comprise one, two or more species of resin selected among various known resins such as, for instance, acrylic resins, rubber-based resins (based on natural rubber, synthetic rubber, a mixture of these, etc.), silicone-based resins, polyester-based resin, urethane-based resin, polyether-based resins, polyamide-based resins, and fluororesins. Here, the acrylic resin refers to a resin whose primary component (base polymer) is an acrylic polymer. The same applies to the rubber-based resin and other resins.

It is noted that the "base polymer" of the resin refers to the primary component among the polymers in the resin and is not to be construed as being limited to anything other than this. As used herein, the "primary component" refers to the highest-content (most abundant) component by weight among the components included. Thus, for instance, when the resin comprises three or more components, the amount of primary component in the resin can be 34 % by weight or greater.

As used herein, the term "acrylic polymer" refers to a polymer derived from a starting monomer mixture including more than 50 % acrylic monomer by weight, or an acrylic polymer. The acrylic monomer refers to a monomer having at least one (meth)acryloyl group per molecule. As used herein, the term "(meth)acryloyl" comprehensively refers to acryloyl and methacryloyl. Similarly, the term "(meth)acrylate" comprehensively refers to acrylate and methacrylate, and the term "(meth)acryl" comprehensively refers to acryl and methacryl.

### (Acrylic resin)

In some embodiments, as the resin-forming material, an acrylic resin can be preferably used. In particular, the resin disclosed herein can be an acrylic resin comprising an acrylic polymer. Acrylic resins tend to have excellent design freedom and are suitable for forming resins satisfying the above properties.

### (Acrylic polymer)

As the acrylic resin disclosed herein, for instance, a resin comprises, as the base polymer, an acrylic polymer formed from monomers including an alkyl (meth)acrylate having a linear or branched alkyl group with 1 up to 20 carbon atoms at the ester terminus. Hereinafter, an alkyl (meth)acrylate having, at the ester terminus, an alkyl group with X up to Y number of carbon atoms may be referred to as an "C_{X-Y} alkyl (meth)acrylate." For easy balancing of properties, the ratio of C₁₋₂₀ alkyl (meth)acrylate in the entire monomers (all monomers) of the acrylic polymer according to some embodiments is, for instance, 25 % by weight or higher, suitably 30 % by weight or higher, or preferably 35 % by weight or higher. In other embodiments, the ratio of C₁₋₂₀ alkyl (meth)acrylate in all monomers of the acrylic polymer is suitably above 50 % by weight, preferably 70 % by weight or higher, more preferably 80 % by weight or higher, for instance, even 90 % by weight or higher. The ratio of C₁₋₂₀ alkyl (meth)acrylate among the monomers can be, for instance, 99.9 % by weight or lower, 99 % by weight or lower, or even 95 % by weight or lower. From the standpoint of adjusting the acrylic resin's surface hardness, storage modulus, Tg and so on, the ratio of C₁₋₂₀ alkyl (meth)acrylate in all monomers of the acrylic polymer according to some embodiments can be, for instance, 80 % by weight or lower, 60 % by weight or lower, 50 % by weight or lower, (e.g., below 50 % by weight) or even 40 % by weight or lower. For the C₁₋₂₀ alkyl (meth)acrylate, solely one species or a combination of two or more species can be used.

Non-limiting specific examples of the C₁₋₂₀ alkyl (meth)acrylate include methyl (meth)acrylate, ethyl (meth)acrylate, propyl (meth)acrylate, isopropyl (meth)acrylate, n-butyl (meth)acrylate, isobutyl (meth)acrylate, s-butyl (meth)acrylate, t-butyl (meth)acrylate, pentyl (meth)acrylate, isopentyl (meth)acrylate, hexyl (meth)acrylate, heptyl (meth)acrylate, octyl (meth)acrylate, 2-ethylhexyl (meth)acrylate, isooctyl (meth)acrylate, nonyl (meth)acrylate, isononyl (meth)acrylate, decyl (meth)acrylate, isodecyl (meth)acrylate, undecyl (meth)acrylate, dodecyl (meth)acrylate, tridecyl (meth)acrylate, tetradecyl (meth)acrylate, pentadecyl (meth)acrylate, hexadecyl (meth)acrylate, heptadecyl (meth)acrylate, stearyl (meth)acrylate, isostearyl (meth)acrylate, nonadecyl (meth)acrylate, and eicosyl (meth)acrylate.

Among these, it is preferable to use at least a C₄₋₂₀ alkyl (meth)acrylate, more preferable to use at least a C₄₋₁₈ alkyl (meth)acrylate, yet more preferable to use at least a C₄₋₁₂ alkyl (meth)acrylate, or particularly preferable to use at least a C₄₋₁₀ alkyl (meth)acrylate. For example, as the monomer(s), one or each of n-butyl acrylate (BA) and 2-ethylhexyl acrylate (2EHA) is preferably included. Other examples of C₄₋₂₀ alkyl (meth)acrylates that are preferably used include isononyl acrylate, n-butyl methacrylate (BMA), 2-ethylhexyl methacrylate (2EHMA), and isostearyl acrylate (iSTA). For the C₄₋₁₈ alkyl (meth)acrylate, solely one species or a combination of two or more species can be used.

In some embodiments, in the monomers forming the acrylic polymer, the ratio of C₄₋₁₈ alkyl (meth)acrylate (preferably C₄₋₁₂, or more preferably C₄₋₁₀ alkyl (meth)acrylate) in C₁₋₂₀ alkyl (meth)acrylate is preferably 30 % by weight or higher, more preferably 40 % by weight or higher, possibly 50 % by weight or higher, 70 % by weight or higher, for instance, 80 % by weight or higher, 90 % by weight or higher, or even 95 % to 100 % by weight.

In other embodiments, to increase the acrylic resin's Tg and adjust the surface hardness, storage modulus, etc., it is preferable to use an alkyl (meth)acrylate (high-Tg alkyl (meth)acrylate) having a homopolymer glass transition temperature (Tg) of 10 °C or higher among the linear and branched C₁₋₂₀ alkyl (meth)acrylates. Favorable examples of such alkyl (meth)acrylates include methyl methacrylate (MMA) (homopolymer Tg: 105 °C) and BMA (homopolymer Tg: 20 °C). For the high-Tg alkyl (meth)acrylate, solely one species or a combination of two or more species can be used.

In the monomers forming the acrylic polymer, the ratio of the high-Tg alkyl (meth)acrylate in the C₁₋₂₀ alkyl (meth)acrylate is preferably 10 % by weight or higher, more preferably 20 % by weight or higher, yet more preferably 30 % by weight or higher, or possibly 35 % by weight or higher, for instance, 40 % by weight or higher. The ratio of the high-Tg alkyl (meth)acrylate in the C₁₋₂₀ alkyl (meth)acrylate is, for instance, possibly 70 % by weight or lower, preferably 60 % by weight or lower, also possibly 50 % by weight or lower, 30 % by weight or lower, 10 % by weight or lower, or even 3 % by weight or lower. The C₁₋₂₀ alkyl (meth)acrylate can be essentially free of high-Tg alkyl (meth)acrylates.

In addition to the alkyl (meth)acrylate, the monomers forming the acrylic polymer may include, as necessary, another monomer (copolymerizable monomer) that is able to copolymerize with the alkyl (meth)acrylate. As the copolymerizable monomer, it is possible to suitably use a monomer having a polar group (e.g., a carboxy group, a hydroxy group, a nitrogen atom-containing ring, etc.) or a monomer having a relatively high (e.g., 10 °C or higher) homopolymer glass transition temperature. The monomer having a polar group may be useful for introducing a cross-linking point into the acrylic polymer or increasing cohesive strength of the acrylic resin. For the copolymerizable monomer, solely one species or a combination of two or more species can be used.

Non-limiting specific examples of the copolymerizable monomer include carboxy group-containing monomers, acid anhydride group-containing monomers, hydroxy group-containing monomers, sulfonate or phosphate group-containing monomers, epoxy group-containing monomers, cyano group-containing monomers, isocyanate group-containing monomers, amide group-containing monomers, amino group-containing monomers, monomers having N-containing rings, monomers having succinimide structures, maleimides, aminoalkyl (meth)acrylates, alkoxy group-containing monomers, alkoxysilyl group-containing monomers, vinyl esters, vinyl ethers, aromatic vinyl compounds, olefins, (meth)acrylates having alicyclic hydrocarbon groups, (meth)acrylates having aromatic hydrocarbon groups; as well as heteroring-containing (meth)acrylates such as tetrahydrofurfuryl (meth)acrylate, halogen atom-containing (meth)acrylates such as vinyl chloride and fluorine atom-containing (meth)acrylates, silicon atom-containing (meth)acrylates such as silicone (meth)acrylate, and (meth)acrylic esters obtained from terpene compound derivative alcohols. In particular, carboxy group-containing monomers, hydroxy group-containing monomers, monomers having N-containing rings and (meth)acrylates having alicyclic hydrocarbon groups are preferable.

Examples of carboxy group-containing monomers as favorable examples of the copolymerizable monomer include acrylic acid, methacrylic acid, carboxyethyl acrylate, carboxypentyl acrylate, itaconic acid, maleic acid, fumaric acid, crotonic acid and isocrotonic acid.

Examples of hydroxy group-containing monomers include hydroxyalkyl (meth)acrylates such as 2-hydroxyethyl (meth)acrylate, 2-hydroxypropyl (meth)acrylate, 2-hydroxybutyl (meth)acrylate, 3-hydroxypropyl (meth)acrylate, 4-hydroxybutyl (meth)acrylate, 6-hydroxyhexyl (meth)acrylate, 8-hydroxyoctyl (meth)acrylate, 10-hydroxydecyl (meth)acrylate, 12-hydroxyauryl (meth)acrylate and (4-hydroxymethylcyclohexyl)methyl (meth)acrylate.

Examples of monomers having N-containing rings include N-vinyl-2-pyrrolidone, N-methylvinylpyrrolidone, N-vinylpyridine, N-vinylpiperidone, N-vinylpyrimidine, N-vinylpiperazine, N-vinylpyrazine, N-vinylpyrrole, N-vinylimidazole, N-vinyloxazole, N-(meth)acryloyl-2-pyrrolidone, N-(meth)acryloylpiperidine, N-(meth)acryloylpyrrolidine, N-vinylmorpholine, N-vinyl-3-morpholinone, N-vinyl-2-caprolactam, N-vinyl-1,3-oxazin-2-one, N-vinyl-3,5-morpholinedione, N-vinylpyrazole, N-vinylisoxazole, N-vinylthiazole, N-vinylisothiazole and N-vinylpyridazine (e.g., lactams including N-vinyl-2-caprolactam).

Examples of (meth)acrylates having alicyclic hydrocarbon groups include alicyclic hydrocarbon group-containing (meth)acrylates such as cyclopentyl (meth)acrylate, cyclohexyl (meth)acrylate, isobomyl (meth)acrylate, dicyclopentanyl (meth)acrylate, and adamantyl (meth)acrylate.

When using such a polar group-containing monomer, its amount used is not particularly limited. For instance, it is suitably at least 0.01 % by weight of the entire monomers. From the standpoint of obtaining greater effect of the use of the polar group-containing monomer, the amount of polar group-containing monomer used can be 0.1 % by weight or more of the entire monomers, or even 0.5 % by weight or more. For easy balancing of properties (surface hardness, storage moduli, Tg, etc.), the amount of polar group-containing monomer is suitably 60 % by weight or less of the entire monomers, possibly 50 % by weight or less, or even 40 % by weight or less.

When using a high-Tg monomer having a homopolymer Tg of 10 °C or higher, its amount is not particularly limited. For instance, it is suitably 1 % by weight or more of the entire monomers. From the standpoint of better obtaining the effect of the high-Tg monomer use, of the entire monomers, the amount of high-Tg monomer can be 10 % by weight or greater, 30 % by weight or greater, or even 40 % by weight or greater (e.g., 50 % by weight or greater). For easy balancing of properties (surface hardness, storage moduli, Tg, etc.), of the entire monomers, the amount of high-Tg monomer is suitably 80 % by weight or less, preferably 70 % by weight or less, more preferably 60 % by weight or less, possibly 50 % by weight or less, 40 % by weight or less, or even 30 % by weight or less (e.g., 10 % by weight or less). It is noted that needless to say, among high-Tg monomers, some may also be considered polar group-containing monomers, and vice versa.

In some embodiments, the monomers forming the acrylic polymer may include a hydroxy group-containing monomer. With the use of hydroxy group-containing monomer, the acrylic resin's cohesive strength and crosslinking degree (e.g., crosslinking by an isocyanate crosslinking agent) can be favorably adjusted. As the hydroxy group-containing monomer, the examples listed earlier and the like can be used. For instance, 2-hydroxyethyl acrylate (HEA) and 4-hydroxybutyl acrylate (4HBA) can be preferably used. For the hydroxy group-containing monomer, solely one species or a combination of two or more species can be used.

When using a hydroxy group-containing monomer, its amount used is not particularly limited. In some preferable embodiments, of the entire monomers, the amount of hydroxy group-containing monomer is 0.01 % by weight or greater, suitably 0.1 % by weight or greater, preferably 0.5 % by weight or greater, or more preferably 1 % by weight or greater. In some embodiments, the amount of hydroxy group-containing monomer is, for instance, suitably 10 % by weight or less, possibly 5 % by weight or less, 3 % by weight or less, or even 1 % by weight or less.

In some embodiments, the monomers forming the acrylic polymer may include an alicyclic hydrocarbon group-containing (meth)acrylate. This can increase the acrylic resin's cohesive strength and Tg. As the alicyclic hydrocarbon group-containing (meth)acrylate, the aforementioned examples and the like can be used. For instance, cyclohexyl acrylate (CHA) and isobomyl acrylate (IBXA) can be preferably used. For the alicyclic hydrocarbon group-containing (meth)acrylate, solely one species or a combination of two or more species can be used.

When using an alicyclic hydrocarbon group-containing (meth)acrylate, its amount used is not particularly limited. For instance, of the entire monomers, it can be 1 % by weight or greater, or even 10 % by weight or greater. In some preferable embodiments, of the entire monomers, the amount of alicyclic hydrocarbon group-containing (meth)acrylate is 30 % by weight or greater, or more preferably 40 % by weight or greater, for instance, possibly 50 % by weight or greater. For easy balancing of properties (surface hardness, storage moduli, Tg, etc.), of the entire monomers, the maximum amount of alicyclic hydrocarbon group-containing (meth)acrylate is suitably 80 % by weight or less, preferably 70 % by weight or less, more preferably 60 % by weight or less, possibly 50 % by weight or less, 40 % by weight or less, or even 30 % by weight or less (e.g., 10 % by weight or less). In some embodiments, the ratio of the alicyclic hydrocarbon group-containing (meth)acrylate in the monomers forming the acrylic polymer can be 3 % by weight or less, or even 1 % by weight or less. The monomers can also be essentially free of alicyclic hydrocarbon group-containing (meth)acrylates.

In some preferable embodiments, the monomers forming the acrylic polymer may include a monomer having a nitrogen atom (N). This can increase the acrylic resin's cohesive strength. As the nitrogen atom-containing monomer, the examples listed earlier and the like can be used. A favorable example of the nitrogen atom-containing monomer is a monomer having a N-containing ring. Examples include cyclic N-vinyl amides. In particular, N-vinyl-2-pyrrolidone (NVP) can be preferably used. Other favorable examples of the nitrogen atom-containing monomer include (meth)acrylamides. For the N-containing monomer, solely one species or a combination of two or more species can be used.

The amount of N-containing monomer (preferably a monomer having a N-containing ring) is not particularly limited. For instance, it can be 1 % by weight or more of all monomers, 3 % by weight or more, or even 5 % by weight or more. Of all monomers, the amount of N-containing monomer is, for instance, suitably 30 % by weight or less, 15 % by weight or less, or even 10 % by weight or less. In some embodiments, the ratio of the N-containing monomer in the monomers forming the acrylic polymer can be 3 % by weight or less, or even 1 % by weight or less. The monomers can also be essentially free of N-containing monomers.

In some embodiments, the ratio of carboxy group-containing monomer in the monomers of the acrylic polymer can be, for instance, 0 % by weight or greater and below 10 % by weight, below 3 % by weight, or even below 1 % by weight (e.g., below 0.1 % by weight). Carboxy group-containing monomers may not be substantially used as the monomers of the acrylic polymer. Here, that certain components are "not substantially used" means that these components are not used at least intentionally.

In the polymerization, a known or commonly used thermal polymerization initiator or photopolymerization initiator can be used in accordance with the polymerization method and polymerization conditions. These polymerization initiators can be used solely as one species or in a combination of two or more species.

The thermal polymerization initiator is not particularly limited. For example, azo-based polymerization initiator, peroxide-based polymerization initiator, a redox-based polymerization initiator by combination of a peroxide and a reducing agent, substituted ethane-based polymerization initiator and the like can be used. More specific examples include, but not limited to, azo-based initiators such as 2,2'-azobisisobutyronitrile (AIBN), 2,2'-azobis(2-methylpropionamidine) disulfate, 2,2'-azobis(2-amidinopropane) dihydrochloride, 2,2'-azobis[2-(5-methyl-2-imidazolin-2-yl)propane] dihydrochloride, 2,2'-azobis(N,N'-dimethyleneisobutylamidine), and 2,2'-azobis[N-(2-carboxyethyl)-2-methylpropionamidine] tetrahydrate (VA-057); persulfates such as potassium persulfate and ammonium persulfate; peroxide-based initiators such as benzoyl peroxide, t-butyl hydroperoxide, and hydrogen peroxide; substituted ethane-based initiators such as phenyl-substituted ethane; redox-based initiators such as combination of a persulfate salt and sodium hydrogen sulfite, and combination of a peroxide and sodium ascorbate. Thermal polymerization can be preferably carried out at a temperature of, for instance, about 20 °C to 100 °C (typically 40 °C to 80 °C).

The photopolymerization initiator is not particularly limited. It is possible to use, for instance, ketal-based photopolymerization initiators, acetophenone-based photopolymerization initiators, benzoin ether-based photopolymerization initiators, acylphosphine oxide-based photopolymerization initiators, α-ketol photopolymerization initiators, aromatic sulphonyl chloride-based photopolymerization initiators, photoactive oxime-based photopolymerization initiators, benzoin-based photopolymerization initiators, benzylic photopolymerization initiators, benzophenone-based photopolymerization initiators, and thioxanthone-based photopolymerization initiators.

Such thermal polymerization initiator or photopolymerization initiator can be used in a usual amount in accordance with the polymerization method, embodiment of polymerization, etc., and there are no particular limitations to the amount. For instance, relative to 100 parts by weight of monomers to be polymerized, about 0.001 part to 5 parts by weight (typically about 0.01 part to 2 parts by weight, e.g., about 0.01 part to 1 part by weight) of polymerization initiator can be used.

In the polymerization, various kinds of heretofore known chain transfer agent (which may also be thought as molecular weight-adjusting agent or polymerization degree-adjusting agent) can be used as necessary. As the chain transfer agent, mercaptans can be preferably used, such as n-dodecyl mercaptan, t-dodecyl mercaptan, thioglycolic acid and α-thioglycerol. Alternatively, a chain transfer agent free of sulfur atoms (a sulfur-free chain transfer agent) can be used as well. For the chain transfer agent, solely one species or a combination of two or more species can be used. When using a chain transfer agent, it can be used in an amount of, for instance, about 0.01 part to 1 part by weight to 100 parts by weight of the monomers. The art disclosed herein can also be preferably implemented in an embodiment that uses no chain transfer agent.

The resin disclosed herein can be formed using a resin composition that includes monomers having an aforementioned composition in a polymer form, in a non-polymerized form (i.e., a form where polymerizable functional groups are unreacted), or as a mixture of these. The resin composition may exist in various forms such as a water-dispersed resin composition in which the resin (resin component) is dispersed in water, a solvent-based resin composition containing the resin in an organic solvent, an active energy ray-curable resin composition prepared to form resin when cured by active energy rays such as UV rays and radioactive rays, a hot-melt resin composition that is applied in a thermally melted state and forms resin when cooled to near room temperature. The resin composition according to some embodiments can be a solvent-based resin composition or a solvent-free resin composition. The solvent-free resin composition encompasses an active energy ray-curable resin composition and a hot-melt resin composition.

The resin composition according to some embodiments can be an active energy ray-curable resin composition. The term "active energy ray" in this Description refers to an energy ray having energy capable of causing a chemical reaction such as polymerization, crosslinking, initiator decomposition, etc. Examples of the active energy ray herein include lights such as ultraviolet (UV) rays, visible lights, infrared lights, radioactive rays such as α rays, β rays, γ rays, electron beam, neutron radiation, and X rays. A favorable example of the active energy ray-curable resin composition is a photocurable resin composition. The photocurable resin composition has an advantage of being able to easily form even a thick resin sheet (resin layer). In particular, a UV ray-curable resin composition is preferable.

The photocurable resin composition typically comprises at least some of the monomers used to form the composition (possibly a certain species among the monomers or a fraction of its quantity) as a polymer. The polymerization method for forming the polymer is not particularly limited. Heretofore known various polymerization methods can be suitably used, for instance, thermal polymerization (typically carried out in the presence of a thermal polymerization initiator) such as solution polymerization, emulsion polymerization, and bulk polymerization; photopolymerization carried out by irradiating light such as UV ray, etc. (typically in the presence of a photopolymerization initiator); and radioactive ray polymerization carried out by irradiating radioactive rays such as β rays and γ rays. In particular, photopolymerization is preferable.

The photocurable resin composition according to some preferable embodiments comprises a partial polymer (partial polymerization product, e.g., a partial acrylic polymer) of the monomers. Such a partial polymer is typically a mixture of a polymer derived from the monomers and unreacted monomers, and it preferably has a syrup form (viscous liquid). Hereinafter, a partial polymer having such a form may be referred to as "monomer syrup" or simply "syrup." The polymerization method for partial polymerization of the monomers is not particularly limited. Various polymerization methods such as those described earlier can be suitably selected and used. From the standpoint of the efficiency and convenience, a photopolymerization method can be preferably used. Photopolymerization allows easy control of the monomer conversion of the monomers through the polymerization conditions such as the radiation dose (amount) of light.

The monomer conversion of the monomer mixture in the partial polymer is not particularly limited. The monomer conversion can be, for instance, about 70 % by weight or less. From the standpoint of the ease of preparing the resin composition containing the partial polymer, the ease of coating and moldability, etc., it is suitably about 50 % by weight or less, or preferably about 40 % by weight or less. The minimum monomer conversion is not particularly limited. It is about 1 % by weight or greater, or usually suitably about 5 % by weight or greater.

The partial polymer-containing resin composition may include other components (e.g., photopolymerization initiator as well as polyfunctional monomer, crosslinking agent and the like described later) used as necessary. The method of adding such other components is not particularly limited. For instance, it can be included in the monomer mixture in advance or added to the partial polymer as well.

The resin according to some embodiments is formed from a water-dispersed resin composition. Typical examples of the water-dispersed resin composition include an emulsion-based resin composition. The emulsion-based resin composition typically comprises a polymerization product of monomers and an additive used as necessary. Emulsion polymerization of the monomers is usually carried out in the presence of an emulsifier. By emulsion polymerization, a polymerization reaction mixture is obtained as an emulsion in which the polymerization product (polymer) of the monomers is dispersed in water. The water-dispersed resin composition used for forming the resin can be preferably produced using the polymerization reaction mixture.

The emulsifier used in the emulsion polymerization is not particularly limited; known anionic emulsifiers, nonionic emulsifiers and the like can be used. These emulsifiers can be used singly as one species or in a combination of two or more species. Non-limiting examples of anionic emulsifiers include sodium lauryl sulfate, ammonium lauryl sulfate, sodium dodecyl benzene sulfonate, sodium polyoxyethylene lauryl sulfate, sodium polyoxyethylene alkyl ether sulfates, ammonium polyoxyethylene alkyl phenyl ether sulfates, sodium polyoxyethylene alkyl phenyl ether sulfates, and sodium polyoxyethylene alkyl sulfosuccinates. Non-limiting examples of non-ionic emulsifiers include polyoxyethylene alkyl ethers, polyoxyethylene alkyl phenyl ethers, polyoxyethylene aliphatic acid esters, and polyoxyethylene-polyoxypropylene block polymers. Reactive functional group-containing emulsifiers (reactive emulsifiers) can be used as well. Examples of reactive emulsifiers include a radically polymerizable emulsifier having a structure of an aforementioned anionic emulsifier or nonionic emulsifier with a radically polymerizable group such as propenyl group and ally ether group introduced therein.

In the emulsion polymerization, the emulsifier can be used in an amount of, for instance, 0.2 part by weight or greater, 0.5 part by weight or greater, or 1.0 part by weight or greater, or even 1.5 parts by weight or greater, relative to 100 parts by weight of the monomers. From the standpoint of enhancing the water resistance or increasing the resin's transparency, in some embodiments, the amount of emulsifier used is usually suitably 20 parts by weight or less to 100 parts by weight of the monomers, preferably 15 parts by weight or less, or possibly even 10 parts by weight or less.

The resin composition according to some embodiments can be a solvent-based resin composition. The solvent-based resin composition typically includes a solution polymerization product of the monomers and additives (e.g., hydrophilicity enhancer) used as necessary. The solvent (polymerization solvent) used for the solution polymerization can be suitably selected among heretofore known organic solvents (e.g., toluene, ethyl acetate, etc.). Solution polymerization gives the polymerization reaction mixture in a form where the polymerization product of the monomers is dissolved in a polymerization solvent. The solvent-based resin composition disclosed herein can be preferably produced using the polymerization reaction mixture.

### (Polyfunctional monomer)

In the resin composition (and further in the resin), a polyfunctional monomer may be used as necessary. The polyfunctional monomer may be helpful for purposes such as adjusting the cohesive strength. During the resin formation, when allowed to react with the ethylenically unsaturated group by irradiation of light (e.g., UV light), etc., the polyfunctional monomer may form a crosslinking structure having suitable flexibility. Accordingly, "polyfunctional monomer" here can be called "crosslinking agent" as well. For instance, it is preferable to use a polyfunctional monomer in a resin formed from a photo-curable resin composition. As the polyfunctional monomer, a compound having two or more ethylenically unsaturated groups can be used. For the polyfunctional monomer, solely one species or a combination of two or more species can be used.

Examples of the ethylenically unsaturated group that the polyfunctional monomer has include, but are not limited to acryloyl group, methacryloyl group, vinyl group and allyl group. Preferable ethylenically unsaturated groups in view of the photoreactivity include acryloyl group and methacryloyl group. In particular, acryloyl group is preferable.

Examples of the polyfunctional monomer include ethylene glycol di(meth)acrylate, propylene glycol di(meth)acrylate, polyethylene glycol di(meth)acrylate, polypropylene glycol di(meth)acrylate, neopentyl glycol di(meth)acrylate, pentaerythritol di(meth)acrylate, pentaerythritol tri(meth)acrylate, dipentaerythritol hexa(meth)acrylate, ethyleneglycol di(meth)acrylate, 1,6-hexanediol di(meth)acrylate, 1,12-dodecanediol di(meth)acrylate, trimethylolpropane tri(meth)acrylate, tetramethylolmethane tri(meth)acrylate, allyl (meth)acrylate, vinyl (meth)acrylate, divinylbenzene, epoxy acrylate, polyester acrylate, urethane acrylate, butyldiol (meth)acrylate and hexyldiol di(meth)acrylate. Among them, trimethylolpropane tri(meth)acrylate, 1,6-hexanediol di(meth)acrylate and dipentaerythritol hexa(meth)acrylate are favorable.

The amount of polyfunctional monomer used depends on its molecular weight, the number of functional groups therein, etc. For instance, it is suitably in the range of about 0.01 part to 3.0 parts by weight to 100 parts by weight of the monomers forming the polymer (typically an acrylic polymer or the monomers thereof) in the resin. In some embodiments, the amount of polyfunctional monomer used to 100 parts by weight of the monomers can be, for instance, 0.02 part by weight or greater, or even 0.1 part by weight or greater. In some embodiments, the amount of polyfunctional monomer used to 100 parts by weight of the monomers is, for instance, suitably 1.0 part by weight or less, or preferably 0.5 part by weight or less. The resin's surface hardness and storage modulus can be adjusted through setting of amounts of polyfunctional monomers.

### (Crosslinking agent)

The resin composition disclosed herein can include a crosslinking agent as necessary, mainly for crosslinking within the resin (layer) or between the resin and the adjacent surface. The type of crosslinking agent is not particularly limited and can be selected among heretofore known crosslinking agents so that, for instance, the crosslinking agent provides suitable crosslinking within the resin layer in accordance with the composition of the resin composition. Examples of the crosslinking agent that can be used include isocyanate-based crosslinking agent, epoxy-based crosslinking agent, oxazoline-based crosslinking agent, aziridine-based crosslinking agent, carbodiimide-based crosslinking agent, melamine-based crosslinking agent, urea-based crosslinking agent, metal alkoxide-based crosslinking agent, metal chelate-based crosslinking agent, metal salt-based crosslinking agents, hydrazine-based crosslinking agent, and amine-based crosslinking agent. These can be used solely as one species or in a combination of two or more species.

The crosslinking agent content (when two or more crosslinking agents are included, the total amount thereof) is not particularly limited. From the standpoint of obtaining a resin having target surface hardness and adhesive properties, the crosslinking agent content is suitably about 5 parts by weight or less to 100 parts by weight of the monomers forming the polymer (e.g., an acrylic polymer or the monomers thereof) in the resin layer, preferably about 0.001 part to 5 parts by weight, more preferably about 0.001 part to 4 parts by weight, or yet more preferably about 0.001 part to 3 parts by weight. Alternatively, the resin composition can also be free of aforementioned crosslinking agents. When using a photocurable resin composition as the resin composition disclosed herein, the resin composition can be essentially free of a crosslinking agent such as an isocyanate-based crosslinking agent. Here, that the resin composition is essentially free of a crosslinking agent means that the amount of crosslinking agent relative to 100 parts by weight of the monomers is less than 0.05 part by weight (e.g., less than 0.01 part by weight).

### (Surfactant)

In some preferable embodiments, the resin composition may comprise a surfactant. The resin (typically a resin sheet) disclosed herein can be porous (favorably including open cells (connected pores)). Thus, in forming a resin with such a porous structure (favorably an open-cell structure), the surfactant can be used for pore diameter adjustment, pore stability, etc. Thus, surfactants used for these purposes are also called foam control agents or foam stabilizers. Examples of surfactants used include ionic surfactants (anionic, cationic, nonionic, and amphoteric surfactants), hydrocarbon-based surfactants, silicone-based surfactants and fluorine-based surfactants. For the surfactant, solely one species or a combination of two or more species can be used. For instance, in water-dispersed resin compositions, in addition to the aforementioned emulsifiers (anionic and nonionic surfactants), amphoteric surfactants such as carboxybetaine can be preferably used.

When the resin composition comprises a surfactant as the foam control agent, the amount of the surfactant used to 100 parts by weight of the monomers forming the polymer (e.g., an acrylic polymer or the monomers thereof) in the resin is, for instance, possibly 0.1 part by weight or greater, 0.5 part by weight or greater, or possibly 1.0 part by weight or greater. The amount of the surfactant used to 100 parts by weight of the monomers is suitably 10 parts by weight or less, preferably 8 parts by weight or less, or even 5 parts by weight or less.

### (Other components)

As necessary, the resin disclosed herein may include, as optional components, various additives such as viscosity-adjusting agent (e.g., thickener), pH-adjusting agent, leveling agent, foam regulator, tackifier resin, crosslinking auxiliary agent, plasticizer, filler, colorant including pigment and dye, etc., stabilizing agent, preservative, anti-aging agent, and so on. With respect to these various additives, those heretofore known can be used according to typical methods. Since these do not particularly characterize the present invention, details are omitted.

In the art disclosed herein, the amounts of non-base-polymer components (other components besides the base polymer) in the resin (favorably an acrylic polymer) may be limited. In the art disclosed herein, the amounts of non-base-polymer components in the resin is, for instance, about 30 % by weight or less, suitably about 10 % by weight or less, preferably about 5 % by weight or less, more preferably about 3 % by weight or less, or possibly about 1.5 % by weight or less (e.g., below 1 % by weight). The composition having such limited amounts of other components besides the base polymer (e.g., an acrylic polymer) can be preferably employed for the photocurable resin composition.

### (Resin formation)

When the resin is in sheet (resin sheet) form, the resin sheet can be formed by providing (e.g., applying) the resin composition to a suitable surface and then subjecting it to a suitable curing process. When two or more different curing processes (drying, crosslinking, polymerization, etc.) are carried out, these can be done at the same time or in stages. When a partial polymer (acrylic monomer syrup) of the monomers is used for the resin composition, final copolymerization reaction is typically carried out as the curing process. That is, the partial polymer is subjected to a further copolymerization reaction to form a fully polymerized product. For instance, with a photocurable resin composition, photoirradiation is carried out. As necessary, curing treatment such as crosslinking and drying can also be carried out. For instance, when drying is necessary with a photocurable resin composition (e.g., in case of a photocurable resin composition in a form of partial polymer of monomers dissolved in an organic solvent), photoirradiation can be carried out after the composition is allowed to dry. With respect to a resin composition using a fully polymerized product, processes such as drying (drying with heat) and crosslinking are typically carried out as necessary as the curing treatment.

The resin composition can be applied with, for example, a conventional coater such as a gravure roll coater, a reverse roll coater, a kiss-roll coater, a dip roll coater, a bar coater, a knife coater and a spray coater.

The resin sheet disclosed herein typically has a monolayer structure. It can have a multilayer structure including one, two or more additional layers besides the resin (layer) as far as the effect of the PSA sheet resin disclosed herein is not impaired. Such an additional layer can be, for instance, a substrate layer to support the resin layer. Such additional layers are not particularly limited. Examples include nonwoven and woven fabrics as well as other porous films. When using nonwoven and woven fabrics, their materials are not particularly limited. It is possible to use materials formed from one, two or more species among natural fibers such as cotton, hemp, and wool; cellulose-based fibers such as rayon and acetate; polyamide fibers such as vinylon and nylon; polyolefin fibers such as polyethylene and polypropylene; polyester fibers such as polyethylene terephthalate (PET); synthetic fibers such as polyurethane fibers; and the like. Porous films include porous resin films formed of polypropylene, polyethylene, PET, polyurethane, etc. In particular, nonwoven fabrics are preferable and nonwoven fabrics formed from synthetic fabrics such as polyester fabric are more preferable. These substrate materials have suitable breathability and moisture permeability, and can also have suitable elasticity/non-elasticity. Thus, for instance, they can be preferably used for applications of adhesion to human skin.

### (Thickness of resin sheet (resin layer))

In an embodiment where the resin disclosed herein is in a sheet form, the resin sheet thickness is not particularly limited and can be, for instance, about 3 µm to 2000 µm. From the standpoint of the ease of handling and processing, etc., in some embodiments, the resin sheet thickness is, for instance, suitably 10 µm or greater, preferably 50 µm or greater, more preferably 70 µm or greater, or yet more preferably 90 µm or greater. A thick resin sheet can effectively exhibit its properties (hardness and viscoelastic properties). The resin sheet thickness is, for instance, possibly 1000 µm or less, suitably 500 µm or less, preferably 300 µm or less, or possibly even 200 µm or less. The resin sheet with a limited thickness is suitable for applications where reduction in weight and thickness is desirable. In some preferable embodiments, the resin sheet thickness is, for instance, 150 µm or less, more preferably 100 µm or less, yet more preferably 70 µm or less, or possibly even 50 µm or less (e.g., 40 µm or less). PSA sheets using thin resin sheets have excellent adherend-conformability and repulsion resistance. For instance, even in long-term adhesion, they tend to be less susceptible to lifting and peeling off from the adherend (e.g., human skin).

### (Porous resin)

In some preferable embodiments, the resin (typically a resin sheet) is porous. A PSA sheet having the resin disclosed herein is preferably used in embodiments adhered to human skin and is desirably moisture-permeable to allow moisture such as sweat to escape when adhered to human skin. By using a porous resin, moisture permeability can be preferably obtained, making it less susceptible to the occurrence of wetness and itchiness caused by sweat, etc. The porous resin preferably comprises open cells. The use of an open-cell resin helps obtain excellent moisture permeability.

The method for forming a porous structure (favorably an open-cell structure) in the resin is not particularly limited. A known foaming method can be suitably used. For instance, in a possible method, a resin composition is mechanically foamed and then cured by drying or the like to form a bubble-containing resin. In particular, examples include a method where under suitable conditions (high pressure, etc.), a bubble-forming gas such as air, nitrogen or carbon dioxide is mixed into the resin composition and the resin composition mixed with the bubble-forming gas is cured to form a bubble-containing resin. Alternatively, an open-cell-containing porous resin can be obtained as follows: A suitable amount of water and a surfactant as necessary are added to a resin composition; after operations such as stirring, the water is evaporated by heating to obtain a porous resin comprising open cells. The foaming device or bubble-mixing device is not particularly limited. For instance, a high-speed stirring device (also emulsifying device or dispersing device) capable of high-speed shearing is preferably used. Conditions for bubble formation, heating and so on can be set according to the target bubble size and shape, the species of bubble-generating component, etc.

### <PSA sheet>

The PSA sheet disclosed herein can be an adhesively single-faced PSA sheet (single-faced PSA sheet) or an adhesively double-faced PSA sheet (double-faced PSA sheet). The PSA sheet typically has a layer A formed of the aforementioned PSA sheet resin. The PSA sheet may have a layer B in addition to the layer A. In other words, the PSA sheet may have a laminated structure of layer A and layer B. The PSA sheet may have a layer C in addition to the layer A. In other words, the PSA sheet may have a laminated structure of layer A and layer C. Furthermore, the PSA sheet may have layers B, A and C in this order. In other embodiments, the PSA sheet may have a layer B constituting the adhesive face of the PSA sheet. The PSA sheet may have a layer A or B and a layer C. The concept of PSA sheet herein may encompass so-called PSA tape, PSA labels, PSA film, etc. The PSA sheet disclosed herein can be in a roll or in a flat sheet. Alternatively, the PSA sheet may be processed into various shapes.

### (Constitutional examples)

The PSA sheet disclosed herein may have, for instance, a cross-sectional structure schematically illustrated in Fig. 2. PSA sheet 1 has a laminated structure of layer A 10 and layer B 20. The first surface 1A of PSA sheet 1 is an application surface (adhesive face) to an adherend and is formed of layer B 20. The second surface 1B (the opposite side of the first surface 1A) of PSA sheet is the backside of adhesive face 1A and is formed of layer A 10. In PSA sheet 1, layer B 20 is a PSA layer whose surface 20Ais an application surface (adhesive face) to an adherend. Layer A 10 is a support layer laminated to the second surface 20B of layer B 20, supporting layer B 20. Layer A 10 and layer B 20 are fixedly joined. PSA sheet 1 prior to use (before applied to an adherend) can be in the form of release-linered PSA sheet 100 as shown in Fig. 2, for example, with adhesive face 1A protected with release liner 41 in which at least the adhesive face side is a releasable surface (release face). Although not shown in particular, the backside 1B of PSA sheet 1 may also be protected with a release liner. While no particular limitations are imposed, when the layer A has a nonwoven fabric or other substrate, the layer B is preferably laminated to the resin layer (the layer A's resin layer side surface) where the substrate is not placed.

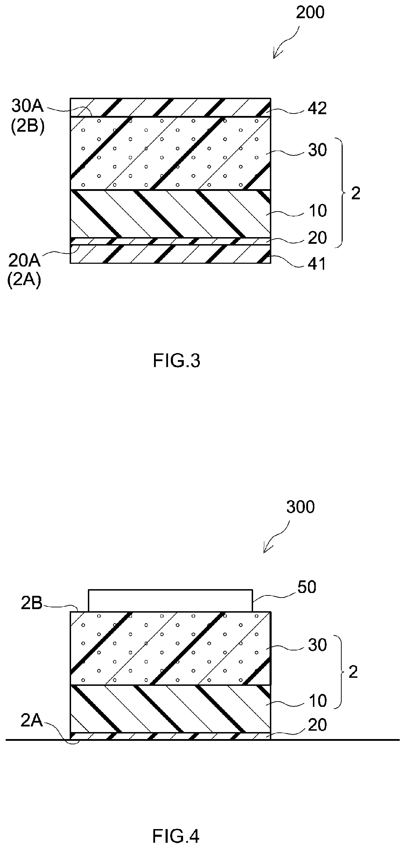

The PSA sheet disclosed herein may be formed as a PSA sheet 2 shown in Fig. 3, for example. PSA sheet 2 shown in Fig. 3 has a layer C 30 in addition to layer A 10 and layer B 20, and has a structure in which layers B 20, A 10 and C 30 are laminated in this order. Layer C 30 is a viscoelastic layer and it is a PSA layer in this embodiment. PSA sheet 2 includes layer A 10 as a middle layer and is formed as an adhesively double-faced PSA sheet (double-faced PSA sheet) in which the respective faces (both non-releasable) of layer A 10 as the middle layer are provided with layers B 20 and C 30 as PSA layers, respectively. The surface 20 A of layer B 20 and the surface 30A of layer C 30 constitute adhesive faces 2A and 2B of PSA sheet 2, respectively. PSA sheet 2 prior to use can be in the form of release-linered PSA sheet 200, protected with two release liners 41 and 42 of which at least their adhesive face sides are release faces. Alternatively, it can be in the form such that with the release liner 4 1's backside (the surface on the opposite side of the adhesive face 2A) being a release face, adhesive faces 2A and 2B are protected by winding or layering the sheet so that the release liner 4 1's backside comes into contact with adhesive face 2B. It is noted that while no particular limitations are imposed, when the layer A has a nonwoven or other substrate, the layer B is preferably laminated to the resin layer (the layer A's resin layer side surface) where the substrate is not placed.

As for the release liner, no particular limitations are imposed. For example, it is possible to use a release liner in which a surface of a liner substrate such as resin film or paper is release-treated, or a release liner formed from a low adhesive material such as fluorine-based polymer (polytetrafluoroethylene, etc.) and polyolefin-based resin (polyethylene, polypropylene, etc.). For the release treatment, for instance, a release agent such as silicone-based and long-chain alkyl-based release agents can be used. In some embodiments, releasetreated resin film can be preferably used as the release liner.

The PSA sheet disclosed herein may optionally have additional layers (e.g., a substrate layer, primer layer, anchor layer, etc.) different from the layers A, B and C while they do not impair the effects of the invention.

### <Layer A>

The layer A forming the PSA sheet is formed of the aforementioned PSA sheet resin, in particular, a sheet of the PSA sheet resin (resin sheet). The layer A is characterized by having a storage modulus at 25 °C in the range of 10 MPa to 500 MPa and a storage modulus at 37 °C in the range of 0.5 MPa to 20 MPa. With the layer A's 25°C and 37°C storage moduli in the respective numerical ranges, the PSA sheet gently conforms to the adherend whereby peel resistance and displacement resistance can be obtained. When the PSA sheet is removed from the adherend, the PSA sheet suitably resists deformation during removal and reduces the local concentration of stress on the adherend. Thus, removal can be completed while preventing adherend damage caused by the stress. The layer A's possible 25°C and 37°C storage moduli ranges are the same as the ranges described for the PSA sheet resin's 25°C and 37°C storage moduli, so the description is not repeated. Also, the details (properties, structure, materials, composition, formation method, thickness, etc.) of the layer A disclosed herein are the same as those for the PSA sheet resin, so the description is not repeated.

### <Layer B>

The layer B disclosed herein is a PSA layer. The species of PSA forming the PSA layer is not particularly limited. The PSA may comprise, as adhesive polymer(s), one, two or more species of various rubber-like polymers such as acrylic polymers, rubber-based polymers (natural rubber, synthetic rubber, mixtures thereof, etc.), polyester-based polymers, urethane-based polymers, polyether-based polymers, silicone-based polymers, polyamide-based polymers and fluoropolymers. In view of adhesive properties, cost and so on, it is preferable to use a PSA comprising an acrylic polymer or rubber-based polymer as base polymer. In particular, a PSA (acrylic PSA) comprising an acrylic polymer as base polymer is preferable. The art disclosed herein is preferably implemented in an embodiment using an acrylic PSA.

It is noted that the "base polymer" of a PSA refers to the primary component (e.g., a component accounting for more than 50 % by weight) of the polymers (typically polymers that exhibit rubber elasticity in the temperature range near room temperature) in the PSA.

Mainly described below are PSA layers formed of acrylic PSA, in other words, PSA sheets having acrylic PSA layers; however, the PSA layer of the PSA sheet disclosed herein is not to be limited to one formed of an acrylic PSA.

### (Acrylic polymer)

The acrylic PSA layer comprises an acrylic polymer as base polymer. Acrylic polymers offer a high degree of freedom in molecular design and tend to achieve adhesive properties (typically adhesive strength) suitable for the art disclosed herein. One example of a preferable acrylic polymer used in the acrylic PSA is a polymer formed from a starting monomer mixture that comprises an alkyl (meth)acrylate as a primary monomer and may further comprise a secondary monomer copolymerizable with the primary monomer. The primary monomer here refers to a component accounting for more than 50 % by weight of the monomer composition in the starting monomer mixture.

As the alkyl (meth)acrylate, for instance, a C₁₋₂₀ alkyl (meth)acrylate can be preferably used. From the standpoint of improving adhesive properties (adhesive strength, holding power, etc.), the primary monomer is suitably a C₁₋₁₄ alkyl (meth)acrylate, preferably a C₁₋₁₀ alkyl (meth)acrylate, more preferably a C₄₋₈ alkyl (meth)acrylate, or yet more preferably a C₄₋₈ alkyl acrylate. As the C₁₋₂₀ alkyl (meth)acrylate, it is possible to use the same species as the C₁₋₂₀ alkyl (meth)acrylates exemplified as the monomers of the acrylic polymer that can be used in the PSA sheet resin. For the C₁₋₂₀ alkyl (meth)acrylate, solely one species or a combination of two or more species can be used. As the C₁₋₂₀ alkyl (meth)acrylate, BA and 2EHA are preferable, and 2EHA is particularly preferable.

The ratio of C₁₋₂₀ alkyl (meth)acrylate in the monomers forming the acrylic polymer is suitably above 50 % by weight, preferably 60 % by weight or higher (e.g., 65 % by weight or higher), possibly 70 % by weight or higher, 80 % by weight or higher, or even 90 % by weight or higher. The maximum ratio of C₁₋₂₀ alkyl (meth)acrylate is not particularly limited and is suitably 99 % by weight or lower. From the standpoint of effectively obtaining properties (e.g., cohesive strength) based on copolymerizable monomers such as polar group-containing monomers, it is preferably 90 % by weight or lower, more preferably 80 % by weight or lower, or possibly even 75 % by weight or lower.

In some embodiments, in the monomers forming the acrylic polymer, the ratio of C₄₋₁₂ alkyl (meth)acrylate (preferably C₄₋₁₀ alkyl (meth)acrylate, more preferably C₄₋₈ alkyl acrylate, particularly preferably 2EHA) to C₁₋₂₀ alkyl (meth)acrylate is preferably 50 % by weight or higher, or more preferably 80 % by weight or higher, yet more preferably 90 % by weight or higher, or possibly even 95 % to 100 % by weight. Such a monomer composition helps obtain superior adhesive properties.

Along with the alkyl (meth)acrylate, the monomers forming the acrylic polymer preferably include other monomer(s) copolymerizable with the alkyl (meth)acrylate (copolymerizable monomer(s)). By using a suitable copolymerizable monomer, excellent adhesive properties (adhesive strength and holding power) can be preferably obtained. The copolymerizable monomer preferably has a functional group and more preferably has a polar group (e.g., carboxy group, hydroxy group, N-containing ring, etc.). Polar group-containing monomers are useful in introducing crosslinking points into the acrylic polymer and increasing the cohesive strength and adhesive strength of the acrylic PSA. As the copolymerizable monomer, it is possible to use the same species as the copolymerizable monomers exemplified as the monomers of the acrylic polymer that can be used in the PSA sheet resin. In particular, carboxy group-containing monomers, hydroxy group-containing monomers and monomers having N-containing rings are preferable. For the copolymerizable monomer, solely one species or a combination of two or more species can be used.

When the acrylic polymer-forming monomers include a polar group-containing monomer as a copolymerizable monomer, the amount of the polar group-containing monomer in the monomers is not particularly limited. From the standpoint of suitably obtaining the effect of using the polar group-containing monomer, the amount of the polar group-containing monomer in the monomers is, for instance, possibly 0.1 % by weight or greater, suitably 1 % by weight or greater, or possibly even 3 % by weight or greater. From the standpoint of easy balancing of adhesive properties in relation to the primary monomer, the amount of the polar group-containing monomer in the monomers is suitably 50 % by weight or less, preferably 40 % by weight or less, or even 30 % by weight or less.

In some preferable embodiments, an acidic group-containing monomer is used as the monomer copolymerizable with the alkyl (meth)acrylate (primary monomer). With the use of an acidic group-containing monomer, cohesion can be enhanced based on its polarity. When using a crosslinking agent such as an isocyanate-based, epoxy-based or other crosslinking agent, the acid group (typically a carboxy group) serves as a crosslinking point in the acrylic polymer. These effects can preferably combine adhesion to the adherend with holding power.

A preferable acidic group-containing monomer is a carboxy group-containing monomer. As the carboxy group-containing monomer, the aforementioned examples can be used. Favorable examples of the carboxy group-containing monomer include acrylic acid (AA) and methacrylic acid (MAA). In particular, AA is more preferable. As the acidic group-containing monomer, ethylenically unsaturated dicarboxylic acid and its anhydride (maleic anhydride, itaconic anhydride, etc.) can also be used. For the acidic group-containing monomer, solely one species or a combination of two or more species can be used.

The amount of the acidic group-containing monomer (preferably a carboxy group-containing monomer) (i.e., the copolymerization ratio of the acidic group-containing monomer in the acrylic polymer) is not particularly limited. For instance, of the entire monomers, it is possibly 0.1 % by weight or greater, preferably 1.0 % by weight or greater, or more preferably 2.0 % by weight or greater (e.g., 2.5 % by weight or greater). The acidic group-containing monomer used in at least such a prescribed amount can increase the PSA layer's cohesive strength. The amount of the acidic group-containing monomer is suitably, for instance, less than 10 % by weight of the entire monomers. From the standpoint of combining adhesion and holding power, it is preferably less than 8.0 % by weight, or more preferably less than 5.0 % by weight.

In some preferable embodiments, the acrylic polymer-forming monomers may include an N-containing monomer. This can increase the cohesive strength of the PSA. As the N-containing monomer, the aforementioned examples can be used. Favorable examples of the N-containing monomer include a monomer having an N-containing ring. Examples include N-vinyl cyclic amides, among which N-vinyl-2-pyrrolidone (NVP) can be preferably used. For the N-containing monomer, solely one species or a combination of two or more species can be used.

The amount of the N-containing monomer (preferably a monomer having an N-containing ring) is not particularly limited. For instance, of the entire monomers, it is possibly 1 % by weight or greater, suitably 10 % by weight or greater, preferably 15 % by weight or greater, or more preferably 20 % by weight or greater (e.g., 22 % by weight or greater). The amount of the N-containing monomer is, for instance, suitably 50 % by weight or less of the entire monomers, preferably 40 % by weight or less, more preferably 35 % by weight or less, yet more preferably 30 % by weight or less, possibly 20 % by weight or less, or even 10 % by weight or less.

In some preferable embodiments, as the monomers of the acrylic polymer, an acidic group-containing monomer (preferably a carboxy group-containing monomer) and an N-containing monomer (preferably a monomer having an N-containing ring) are used together. This can bring about superior adhesive properties. For instance, even in a thin PSA layer, high adhesive strength can be obtained. While no particular limitations are imposed, it is thought that the copresence of the acidic group-containing monomer and the N-containing monomer brings about high adhesive strength through their acid-base interaction, and this effect is especially effective in a PSA layer with a limited thickness. The ratio (A_{N}/A_{C}) of the amount A_{N} of the N-containing monomer to the amount A_{C} of the acid group-containing monomer is not particularly limited. It is, for instance, possibly 0.1 or higher, suitably 1 or higher, preferably 3 or higher, more preferably 5 or higher, or possibly even 7 or higher. The A_{N}/A_{C} ratio is possibly 20 or lower, suitably 15 or lower, preferably 12 or lower, or possibly even 9 or lower.

In some embodiments, the ratio of a hydroxy group-containing monomer in the acrylic polymer-forming monomers can be, for instance, below 10 % by weight, below 3 % by weight, or even below 1 % by weight (e.g., below 0.1 % by weight). It is not necessary to substantially use a hydroxyl group-containing monomer as a monomer of the acrylic polymer.

In other embodiments, the acrylic polymer-forming monomers may include an alkoxy group-containing monomer. This helps obtain a flexible PSA. As it provides excellent moisture permeability, the resulting constitution is likely to readily permeates water vapor, easily release moisture generated from the adherend (e.g., human skin) to the outside, and even have excellent conformability to the adherend (e.g., human skin) and repulsion resistance. Favorable examples of the alkoxy group-containing monomer include alkoxyalkyl (meth)acrylates such as 2-methoxyethyl (meth)acrylate, 3-methoxypropyl (meth)acrylate, 2-ethoxyethyl (meth)acrylate, propoxyethyl (meth)acrylate, butoxyethyl (meth)acrylate, and ethoxypropyl (meth)acrylate. For the alkoxy group-containing monomer, solely one species or a combination of two or more species can be used. The amount of the alkoxy group-containing monomer is not particularly limited. For instance, of the entire monomers, it is possibly 1 % by weight or greater, suitably 5 % by weight or greater, or preferably 10 % by weight or greater. For instance, of the entire monomers, it is suitably 40 % by weight or less, preferably 30 % by weight or less, or more preferably 20 % by weight or less.

The acrylic polymer may include, as other monomers, polyfunctional monomers having at least two polymerizable functional groups (typically radically polymerizable functional groups) with unsaturated double bonds such as (meth)acryloyl groups and vinyl groups. By using a polyfunctional monomer as a monomer, the PSA layer's cohesive strength can be increased. The polyfunctional monomers can be used as a crosslinking agent. As the polyfunctional monomer, it is possible to use the same species as the polyfunctional monomers that can be used in the PSA sheet resin. For the polyfunctional monomer, solely one species or a combination of two or more species can be used. The amount of the polyfunctional monomer is not particularly limited, and can be suitably selected so as to achieve the purpose of using the polyfunctional monomer. The amount of the polyfunctional monomer can be in the range of about 0.001 % by weight or greater and 3 % by weight or less of the monomers.

The method for obtaining the polymer (e.g., an acrylic polymer) is not particularly limited. Various polymerization methods known as polymer synthesis methods can be suitably employed, such as solution polymerization, emulsion polymerization, bulk polymerization, suspension polymerization and photopolymerization. For instance, solution polymerization can be preferably employed. The polymerization temperature for solution polymerization can be suitably selected according to the species of monomers and solvent used, the species of polymerization initiator, etc. For instance, it can be about 20 °C to 170 °C (typically about 40 °C to 140 °C).

The solvent used for solution polymerization (polymerization solvent) can be suitably selected among heretofore known organic solvents (toluene, ethyl acetate, etc.). As the initiator used for polymerization, it is possible use the same species as those exemplified as the thermal polymerization initiator that can be used in polymerization of the acrylic polymer in the PSA sheet resin. For instance, it is preferable to use an azo-based polymerization initiator such as AIBN or a peroxide-based initiator. For the polymerization initiator, solely one species or a combination of two or more species can be used. The polymerization initiator can be used in a typical amount. For instance, it can be selected in the range of about 0.005 part to 1 part by weight (typically about 0.01 part to 1 part by weight) to 100 parts by weight of the monomers.

### (Crosslinking agent)

The PSA composition used for forming the layer B disclosed herein may include a crosslinking agent as necessary. The species of crosslinking agent is not particularly limited and can be suitably selected and used among heretofore known crosslinking agents. Examples of such crosslinking agents include isocyanate crosslinking agent, epoxy crosslinking agent, oxazoline crosslinking agent, aziridine crosslinking agent, melamine crosslinking agent, peroxide crosslinking agent, and metal chelate crosslinking agent. For the crosslinking agent, solely one species or a combination of two or more species can be used. Among them, from the standpoint of increasing the cohesive strength, isocyanate crosslinking agents and epoxy crosslinking agents are preferably used, and isocyanate crosslinking agents are more preferable.

The amount of the crosslinking agent is not particularly limited. For instance, it can be selected in the range of about 10 parts by weight or less (e.g., about 0.001 part to 10 parts by weight, preferably about 0.01 part to 5 parts by weight) to 100 parts by weight of the base polymer (e.g., an acrylic polymer). In some preferable embodiments, the amount of the crosslinking agent used to 100 parts by weight of the base polymer (e.g., an acrylic polymer) is less than 1 part by weight, possibly less than 0.5 part by weight, or even less than 0.1 part by weight (e.g., 0.05 part by weight). According to the art disclosed herein, by designing the monomer composition of the base polymer, sufficient cohesive strength can be obtained with the use of a small amount of crosslinking agent.

### (Other optional components)

The layer B disclosed herein can optionally include a tackifier resin for increasing adhesive strength or like purpose. As the tackifier resin, it is possible to use one, two or more species selected among tackifier resins such as phenolic tackifier resins, terpene-based tackifier resins, modified terpene-based tackifier resins, rosin-based tackifier resins, hydrocarbon-based tackifier resins, epoxy-based tackifier resins, polyamide-based tackifier resins, elastomer-based tackifier resins, and ketone-based tackifier resins. When the layer B comprises a tackifier resin, the amount of the tackifier resin is not particularly limited. For instance, it can be suitably set in the range of about 1 part to 100 parts by weight to 100 parts by weight of the polymer.

The PSA composition for forming the layer B may include, as necessary, various additives generally used in the field of PSA compositions such as a leveling agent, cross-linking auxiliary agent, plasticizer, softening agent, filler, coloring agent (dye, pigment), antistatic agent, anti-aging agent, UV absorber, antioxidant, photostabilizer, and dispersant. In an embodiment where the layer B is applied to human skin, the layer B may include a component that reduces skin irritation, such as a carboxylic acid ester and an aliphatic acid ester. As for the various additives, heretofore known species can be used by typical methods. As they do not particularly characterize the present invention, their details are omitted.

In the art disclosed herein, the amount of layer B components other than the base polymer (favorably an acrylic polymer) may be limited. In the art disclosed herein, the amount of non-base-polymer components in the layer B is, for instance, about 30 % by weight or less, suitably about 10 % by weight or less, preferably about 5 % by weight or less, more preferably about 3 % by weight or less, or possibly even less than 1.0 % by weight. The effect of the art disclosed herein can be preferably obtained with a composition having a limited amount of components other than the base polymer (e.g., an acrylic polymer).

### (PSA composition)

The layer B disclosed herein may be a PSA layer formed from an aqueous PSA composition, solvent-based PSA composition, hot-melt PSA composition, or active energy ray-curable PSA composition. The aqueous PSA composition refers to a PSA composition that comprises PSA (PSA layer-forming components) in a solvent (aqueous solvent) whose primary component is water. It typically encompasses so-called a water-dispersed PSA composition (a composition in which at least part of the PSA is dispersed in water) and the like. The solvent-based PSA composition refers to a composition comprising PSA in an organic solvent. From the standpoint of favorably obtaining adhesive properties such as adhesive strength, the art disclosed herein is preferably implemented in an embodiment having a PSA layer formed from a solvent-based PSA composition.

### (Formation method)

The layer B (PSA layer) disclosed herein can be formed by a heretofore known method. For instance, it is possible to employ a method where a PSA composition is applied to a releasable surface (release face) and allowed to dry to form a PSA layer. In a layer-A-containing PSA sheet, for instance, it is possible to employ a direct method where a PSA composition is directly provided (typically applied) to the layer A and allowed to dry to form a PSA layer. It is also possible to employ a transfer method where a PSA composition is provided to a releasable surface (release face) and allowed to dry to form a PSA layer on the surface, and the PSA layer is transferred onto a layer A. As the release face, for instance, a release liner surface can be preferably used. The PSA layer disclosed herein is typically formed in a continuous form, but it is not limited to such a form. For instance, the PSA layer may be formed in a regular or random pattern of dots, stripes, etc.

The PSA composition can be applied by using a heretofore known coater such as a gravure roll coater, die coater and bar coater. Alternatively, the PSA composition can be applied by impregnation, curtain coating, etc. From the standpoint of accelerating the crosslinking reaction, improving production efficiency, and so on, it is preferable to dry the PSA composition by heating. The drying temperature is, for instance, possibly about 40 °C to 150 °C, or preferably about 60°C to 130 °C. After dried, the PSA composition can be further aged for adjustment of component migration in the PSA layer (layer B), progress of crosslinking reaction, relaxation of possible strain in the PSA layer, etc.

### (25°C storage modulus)

The storage modulus at 25°C of the layer B disclosed herein is not particularly limited. The layer B's 25°C storage modulus is, for instance, 0.03 MPa or greater, possibly 0.1 MPa or greater, or even 0.3 MPa or greater. From the standpoint of improving adhesive properties (adhesive strength and holding power), the 25°C storage modulus is preferably 1 MPa or greater, more preferably 1.5 MPa or greater, or yet more preferably 2.0 MPa or greater. The 25°C storage modulus can be, for instance, less than 10 MPa, or even less than 5 MPa. The layer B having such a 25°C storage modulus can exhibit high adhesive strength while retaining good holding power. In some preferable embodiments, the layer B has a 25°C storage modulus of 3 MPa or less.

In an embodiment where the PSA sheet disclosed herein comprises layers A and B, the layer B's 25°C storage modulus G'_{B} is preferably less than the layer A's 25°C storage modulus G'_{A}. By this, while providing adhesive strength with the layer B, the layer A's presence can effectively achieve its effects (peel resistance and displacement resistance in adhesive fixation, adherend damage prevention during removal, etc.). The ratio (G'_{A}/G'_{B}) of the layer A's 25°C storage modulus G'_{A} to the layer B's 25°C storage modulus G'_{B} is preferably higher than 1, more preferably 10 or higher, yet more preferably 20 or higher, possibly 30 or higher, or even 40 or higher. The G'_{A}/G'_{B} ratio is suitably 200 or lower, preferably 100 or lower, more preferably 80 or lower, yet more preferably 60 or lower, possibly 45 or lower, or even 30 or lower.

### (Glass transition temperature)

The layer B's glass transition temperature (Tg) is set to produce target adhesive properties and is not limited to a specific range. From the standpoint of the adhesive strength, etc., the layer B has a Tg of, for instance, suitably about 30°C or lower, preferably 20 °C or lower, more preferably 15°C or lower, possibly 0 °C or lower, or even -20°C or lower. From the standpoint of the cohesive strength, etc., the layer B's Tg is suitably -50°C or higher, preferably -30 °C or higher, more preferably -20°C or higher, yet more preferably -10°C or higher, or possibly even 5 °C or higher (e.g., 10°C or higher).

In an embodiment where the PSA sheet disclosed herein has layers A and B, the layer B's Tg_{B} is preferably lower than the layer A's Tg_{A}. By this, while providing adhesive strength with the layer B, the layer A's presence can effectively achieve its effects (peel resistance and displacement resistance in adhesive fixation, adherend damage prevention during removal, etc.). The difference (Tg_{A} - Tg_{B}) between the layer A's Tg_{A} and the layer B's Tg_{B} is preferably 5 °C or greater, more preferably 10 °C or greater, or possibly even 15 °C or greater. The difference (Tg_{A} - Tg_{B}) is suitably 80 °C or less, preferably 60 °C or less, more preferably 40 °C or less, or yet more preferably 25 °C or less.

The layer B's viscoelastic properties (25°C storage modulus and Tg) can be determined by dynamic viscoelastic analysis. In particular, they are determined by the methods described later in Examples. Based on the content of this description, the layer B's viscoelastic properties can be adjusted through selection of PSA components and composition, PSA preparation conditions, etc.

### (Layer B thickness)

The layer B thickness is not particularly limited. The layer B thickness is typically about 300 µm or less, suitably about 100 µm or less, preferably about 30 µm or less, more preferably less than 10 µm, or possibly even 5 µm or less (e.g., 3 µm or less). With such a limited thickness, the layer B disclosed herein can exhibit desired adhesive strength. A PSA layer with a limited thickness can well adopt to demands for thinning and lightening. The minimum layer B thickness is not particularly limited. From the standpoint of adhesion and adherend conformability, it is, for instance, about 0.5 µm or greater, or suitably about 1.0 µm or greater. In some preferable embodiments, the layer B thickness is greater than 1.0 µm, more preferably 1.5 µm or greater, or possibly even about 3 µm or greater. With increasing PSA layer thickness, superior adhesive properties tend to be likely obtained. In other embodiments, the layer B thickness is about 5 µm or greater, preferably about 10 µm or greater, more preferably 15 µm or greater, or yet more preferably 20 µm or greater, for instance, possibly 30 µm or greater. In an embodiment using a highly flexible layer B (PSA), when the layer B has at least a certain thickness, the resulting constitution is likely to have excellent conformability to the adherend (e.g., human skin) and repulsion resistance. In such an embodiment, the layer B thickness can be, for instance, about 80 µm or less, 60 µm or less, or even 45 µm or less.

In an embodiment where the PSA sheet disclosed herein has layers A and B, their thicknesses are suitably selected so as to obtain the effects of the respective layers and are not limited to specific ranges. The ratio (T_{A}/T_{B}) of the layer A thickness T_{A} to the layer B thickness T_{B} is suitably about 1 or higher. From the standpoint of effectively obtaining the effects (peel resistance and displacement resistance in adhesive fixation, adherend damage prevention during removal, etc.) of the layer A's presence, the T_{A}/T_{B} ratio is preferably 5 or higher, more preferably 10 or higher, yet more preferably 30 or higher, or possibly even 40 or higher (e.g., 45 or higher). From the standpoint of bringing about the adhesive strength of the layer B, the T_{A}/T_{B} ratio is suitably 200 or lower, preferably 100 or lower, or more preferably 80 or lower, for instance, possibly 60 or lower. In other embodiments, the T_{A}/T_{B} ratio can be 0. 1or higher, 0.3 or higher, 0.5 or higher, or even 0.7 or higher (e.g., 0.8 or higher). The art disclosed herein can be preferably implemented in an embodiment where the layer B is relatively larger in thickness than the layer A, or in an embodiment where the layer B thickness is about the same as the layer A thickness. For instance, in an embodiment using a highly flexible layer B (PSA layer), the presence of the layer A satisfying the ratio (T_{A}/T_{B}) in an aforementioned range can achieve its effects such as resistance to peeling and displacement in adhesive fixation, and prevention of adherend damage during removal. In such an embodiment, the T_{A}/T_{B} ratio can be, for instance, 50 or lower, 10 or lower, or even 3 or lower (e.g., 1.5 or lower).

### <Layer C>

The layer C disclosed herein has viscoelasticity and thus is a viscoelastic layer. The viscoelastic material referred to herein is a material combining viscous and elastic properties, that is, a material having properties satisfying that the complex modulus of elasticity has a phase angle above zero, but below π/2 (typically, a material having the properties at 25 °C). From the standpoint of the flexibility, etc., a preferable material has properties satisfying a complex modulus of elasticity E^{∗}(1Hz) < 10⁷ dyne/cm² (typically, a material having the property at 25 °C). According to the PSA sheet with the layer C formed of a viscoelastic material, the amount of energy required for removal from adherend increases whereby it can exhibit greater peel-resistance on flexible and moving adherends such as human skin.

While the composition of the layer C exhibits viscoelastic properties in a temperature range around room temperature, there are no particular limitations to the composition. The layer C may comprise one, two or more species selected from various viscoelastic materials such as acrylic viscoelastic materials, rubber-based viscoelastic materials, silicone-based viscoelastic materials, polyester-based viscoelastic materials, urethane-based viscoelastic materials, polyether-based viscoelastic materials, polyamide-based viscoelastic materials, fluorine-based viscoelastic materials, and the like. Herein, the term acrylic viscoelastic material refers to a viscoelastic material comprising an acrylic polymer as the base polymer (primary component among polymers, i.e., a component accounting for more than 50 % by weight of the content). The same applies to the rubber-based and other viscoelastic materials.

The layer C may be an adhesive layer or a non-adhesive layer. The term "adhesive layer" herein refers to a layer having a peel strength of 0.1 N/20mm or greater when measured based on JIS Z0237 (2004), such that with a SUS304 stainless steel plate being an adherend, 30 minutes after press-bonded to the adherend with a 2 kg roller moved back and forth once in a measurement environment at 23 °C, the layer is peeled in the 180° direction at a tensile speed of 300 mm/min. It is also referred to as a PSA layer. The term "non-adhesive layer" refers to a layer that is not considered as the adhesive layer, typically with the peel strength being below 0.1 N/20mm. A layer that does not bond to a SUS304 stainless steel plate when press-bonded to the stainless steel plate with a 2 kg roller moved back and forth once at 23 °C (i.e., a layer that is essentially non-adhesive) is a typical example included in the concept of the non-adhesive layer referred to herein. Although not particularly limited, the art disclosed herein can be preferably implemented in an embodiment comprising a layer C that is also an adhesive layer, that is, an embodiment comprising a layer C as a PSA layer. For instance, in an embodiment of the PSA sheet whose layer A has a layer A on one face and a layer C on the other face, when the layer C is a PSA layer, the PSA sheet is adhesive on both faces.

In some preferable embodiments, the layer C may be a layer comprising an acrylic polymer as base polymer, that is, an acrylic viscoelastic layer. The layer C having such a composition is preferable since the balance between flexibility and cohesiveness can be readily adjusted. The proportion of the acrylic polymer in the layer C is not particularly limited. It is typically 50 % by weight or greater, preferably 70 % by weight or greater, or more preferably 80 % by weight or greater.

As the acrylic polymer, for example, a polymer of starting monomer(s) comprising an alkyl (meth)acrylate as the primary monomer and possibly further comprising a secondary monomer copolymerizable with the primary monomer is preferable. The primary monomer herein refers to a component that accounts for more than 50 % by weight of all the monomers in the starting monomer(s). In typical, the composition of monomers in the starting monomer(s) is approximately equivalent to the composition of monomeric units in the acrylic polymer.

As the alkyl (meth)acrylate, for instance, a C₁₋₂₀ alkyl (meth)acrylate can be preferably used. From the standpoint of the layer C's viscoelastic properties (possibly adhesive properties), the primary monomer is suitably a C₁₋₁₄ alkyl (meth)acrylate, preferably a C₁₋₁₀ alkyl (meth)acrylate, more preferably a C₄₋₈ alkyl (meth)acrylate, or yet more preferably a C₄₋₈ alkyl acrylate. As the C₁₋₂₀ alkyl (meth)acrylate, it is possible to use the same species as the C₁₋₂₀ alkyl (meth)acrylates exemplified as the monomers of the acrylic polymer that can be used in the PSA sheet resin. For the C₁₋₂₀ alkyl (meth)acrylate, solely one species or a combination of two or more species can be used. As the C₁₋₂₀ alkyl (meth)acrylate, BA and 2 EH A are preferable; and 2 EH A is particularly preferable.

Although not particularly limited, the amount of the C₁₋₂₀ alkyl (meth)acrylate can be, for instance, 60 % by weight or more of all the monomers constituting the acrylic polymer, or it is usually preferably 70 % by weight or more, or more preferably 80 % by weight or more (e.g., 90 % by weight or more). From the standpoint of the layer C's cohesive strength, etc., the amount of the C₁₋₂₀ acrylic (meth)acrylate is suitably 99.5 % by weight or less, preferably 99 % by weight or less, or more preferably 95 % by weight or less.

Examples of the secondary monomer include a monomer having a functional group (or a "functional group-containing monomer" hereinafter). Such a functional group-containing monomer can be used to introduce crosslinking points in the acrylic polymer so as to increase the layer C's cohesive strength. As such a functional group-containing monomer, it is possible to use the same species as the functional group-containing monomers among the copolymerizable monomers exemplified as the monomers of the acrylic polymer that can be used in the PSA sheet resin. For the functional group-containing monomer, solely one species or a combination of two or more species can be used.

When a functional group-containing monomer as those described above is used as the secondary monomer, its amount used can be suitably selected so as to obtain desirable cohesive strength, and it is not particularly limited. The amount of the functional group-containing monomer used can be, for instance, 0.5 % by weight or more of all the monomers constituting the acrylic polymer. It is usually suitably 1 % by weight or more, preferably 3 % by weight or more, or more preferably 5 % by weight or more. From the standpoint of combining flexibility and cohesive strength at a good balance, the amount of the functional group-containing monomer is suitably 30 % by weight or less of all the monomers, preferably 25 % by weight or less, or more preferably 20 % by weight or less.

In order to adjust the glass transition temperature (Tg) or to increase the cohesive strength, etc., the starting monomer(s) may comprise secondary monomer(s) other than the aforementioned functional group-containing monomer. Examples of such secondary monomers include the following:
Carboxylic acid vinyl esters such as vinyl acetate, vinyl propionate, vinyl lactate, vinyl pyvalate, vinyl cyclohexanecarboxylate, and vinyl benzoate;
Aromatic vinyl compounds such as styrene, substituted styrenes (α -methylstyrene, etc.), and vinyl toluene;
Aromatic ring-containing (meth)acrylates such as aryl (meth)acrylate (e.g., phenyl (meth)acrylate), aryloxyalkyl (meth)acrylate (e.g., phenoxyethyl (meth)acrylate), and arylalkyl (meth)acrylate (e.g., benzyl (meth)acrylate);
Olefinic monomers such as ethylene, propylene, isoprene, butadiene, and isobutylene;
Chlorine-containing monomers such as vinyl chloride, and vinylidene chloride;

Vinyl ether-based monomers such as methyl vinyl ether, and ethyl vinyl ether; and the like. These can be used singly as one species or in combination of two or more species. The amount of such secondary monomer(s) used can be suitably selected depending on the purpose and application, and is not particularly limited. For example, it is preferably 10 % by weight or less of all the monomers.

The starting monomer(s) may comprise as necessary a polyfunctional monomer for crosslinking, etc. As the polyfunctional monomer, it is possible to use the same species as the polyfunctional monomers that can be used in the PSA sheet resin. For the polyfunctional monomer, solely one species or a combination of two or more species can be used. From the standpoint of the reactivity, etc., it is usually preferable to use a polyfunctional monomer having two or more (typically three or more) acryloyl groups per molecule. When such a polyfunctional monomer is used, its amount used is not particularly limited. From the standpoint of the flexibility of the layer C, it is usually suitably more than 0% by weight and 2 % by weight or less (more preferably 1 % by weight or less) of all the monomers.

The monomer composition of the acrylic polymer can be selected so that the resulting acrylic polymer has a Tg of, for instance, -70 °C or above, but -10 °C or below. From the standpoint of the flexibility, the acrylic polymer has a Tg of suitably -20 °C or below, preferably -30 °C or below, more preferably -40 °C or below, or yet more preferably -50 °C or below. From the standpoint of the cohesive strength of the layer C, the Tg can be -65 °C or above.

Herein, the Tg of the acrylic polymer refers to a value determined by the Fox equation based on the Tg values of homopolymers of the respective monomers constituting the acrylic polymer and weight fractions (copolymerization ratio by weight) of the monomers. Thus, the Tg of an acrylic polymer can be adjusted by suitably modifying the monomer composition (i.e., types and relative amounts of monomers used for the synthesis of the acrylic polymer). As the Tg values of homopolymers, values given in a known document (e.g., Polymer Handbook, 3rd ed., John Wiley & Sons, Inc., 1989) are used.

The acrylic polymer can be prepared by a known or commonly-used polymerization method. As the polymerization method, can be suitably employed, for instance, thermal polymerization (typically carried out in the presence of a thermal polymerization initiator) such as solution polymerization, emulsion polymerization, bulk polymerization, etc.; active energy ray polymerization carried out by irradiating an active energy ray including lights such as UV lights, etc., as well as radioactive rays such as β rays, γ rays, etc.; and the like. Examples of the active energy ray polymerization referred to herein include photopolymerization carried out by irradiating lights such as UV lights (typically carried out in the presence of a photopolymerization initiator) as well as radiation polymerization carried out by irradiating ionizing radiation such as α rays, β rays, γ rays, neutron beams, electron beams, etc. These polymerization methods can be used singly as one type or in combination of two or more different types.

For the polymerization, depending on the polymerization method and embodiment, etc., a known or commonly-used polymerization initiator can be used. As the polymerization initiator, it is possible to use the same species as the examples of polymerization initiators that can be used in the polymerization of acrylic polymer in the PSA sheet resin. For the polymerization initiator, solely one species or a combination of two or more species can be used. For advantages such as shortened polymerization time, etc., a photopolymerization initiator can be preferably used. Such thermal polymerization initiator or photopolymerization initiator can be used in a typical amount in accordance with the polymerization method or embodiment, etc., without particular limitations. For example, to 100 parts by weight of the starting monomer(s), an initiator can be used at 0.001 part to 5 parts by weight (typically, 0.01 part to 2 parts by weight, e.g., 0.01 part to 1 part by weight).

A preferable composition for forming the layer C comprises a partially-polymerized product obtained by partially polymerizing the monomers. Alternatively, the layer-C-forming composition (viscoelastic layer-forming composition) may be in a form comprising an acrylic polymer (e.g., acrylic polymer at 95 % by weight or higher polymer conversion of the monomers) as a fully-polymerized product of the monomers. For instance, it may be in a form of a solvent-based composition comprising such an acrylic polymer in an organic solvent, a water-dispersed composition such that the acrylic polymer is dispersed in an aqueous solvent, etc.

The layer-C-forming composition may comprise a crosslinking agent. As the crosslinking agent, can be used a crosslinking agent commonly known or used in the field of PSA (e.g., acrylic PSA). Examples include epoxy-based crosslinking agents, isocyanate-based crosslinking agents, silicone-based crosslinking agents, oxazoline-based crosslinking agents, aziridine-based crosslinking agents, silane-based crosslinking agents, alkyletherified melamine-based crosslinking agent, metal chelate-based crosslinking agent, and the like. Alternatively, the composition may be essentially free of such a crosslinking agent.

The layer C may comprise a filler. The inclusion of a filler in a layer C can increase the shear strength of the layer C. This may increase the resistance against peeling the PSA sheet from an adherend (peel strength). The use of a filler may suppress excessive deformation of the layer C, allowing for suitable adjustment of the balance between flexibility and cohesive strength of the PSA sheet as a whole.

As the filler, various particulate substances can be used. Examples of a material constituting such a particulate substance include metals such as copper, nickel, aluminum, chromium, iron, stainless steel, etc.; metal oxides such as alumina, zirconia, etc.; carbides such as silicon carbide, boron carbide, nitrogen carbide, etc.; nitrides such as aluminum nitride, silicon nitride, boron nitride, etc.; other inorganic materials such as calcium carbide, calcium carbonate, aluminum hydroxide, glass, silica, etc.; polymers such as a polystyrene, acrylic resin (e.g., poly(methyl methacrylate)), phenol resin, benzoguanamine resin, urea resin, silicone resin, nylon, polyester, polyurethane, polyethylene, polypropylene, polyamide, polyimide, silicone, vinylidene chloride, etc.; and the like. Alternatively, particles of natural materials such as volcanic shirasu, sand and the like can be used. These can be used singly as one species or in combination of two or more species.

The external form of the particulate substance and the particle structure are not particularly limited. The external form of the particulate substance may be globular, flaky, irregularly-shaped, etc. The particle structure of the particulate substance may be, for instance, dense, porous, hollow, etc.

The art disclosed herein may be preferably implemented in an embodiment where the layer C comprises as the filler a particulate substance having a hollow particle structure (or "hollow particles" hereinafter). From the standpoint of the photopolymerizability (polymerizability), hollow particles formed from an inorganic material can be preferably used. Examples of such hollow particles include balloons made of glass such as hollow glass balloons; hollow balloons made of metal compounds such as hollow alumina balloons; and hollow balloons made of ceramics such as hollow ceramic balloons.

As the hollow glass balloon, examples of usable commercial products include trade names GLASS MICRO BALLOON, FUJI BALLOON H-40 and FUJI BALLOON H-35 available from Fuji Silysia Chemical Ltd.; trade names CEL-STAR Z-20, CEL-STAR Z-27, CEL-STAR CZ-31T, CEL-STAR Z-36, CEL-STAR Z-39, CEL-STAR T-36 and CEL-STAR PZ-6000 available from Tokai Kogyo Co. Ltd.; trade names SEILAX FINE BALLOON available from Fine Balloon KK; trade names Q-CEL^{®} 5020, Q-CEL^{®} 7014, SPHERICEL^{®} 110P8, SPHERICEL^{®} 25P45, SPHERICEL^{®} 34P30 and SPHERICEL^{®} 60P18 available from Potters-Ballotini Co., Ltd.; and trade names SUPER BALLOON BA-15 and SUPER BALLOON 732C available from Showa Chemical Industry Co., Ltd.

The average particle diameter of the hollow particles used is not particularly limited. For example, it can be selected from a range of 1 µm to 500 µm, preferably 5 µm to 400 µm, more preferably 10 µm to 300 µm, or yet more preferably 10 µm to 200 µm (e.g., 10 µm to 150 µm). The average particle diameter of the hollow particles is usually suitably 50 % of the thickness of the layer C or smaller, or preferably 30 % or smaller (e.g., 10 % or smaller).

The specific gravity of the hollow particles is not particularly limited. In view of uniform dispersibility and mechanical strength, etc., for instance, it can be selected from a range of 0.1 g/cm³ to 1.8 g/cm³, preferably 0.1 g/cm³ to 1.5 g/cm³, or more preferably 0.1 g/cm³ to 0.5 g/cm³ (e.g., 0.2 g/cm³ to 0.5 g/cm³).

The amount of the hollow particles used is not particularly limited. For instance, it can be about 1 % to 70 % by volume of the total layer C. It is usually suitably about 5 % to 50 % by volume, or preferably about 10 % to 40 % by volume.

The layer C may have bubbles. The inclusion of bubbles in the viscoelastic layer C can increase the cushioning effect of the PSA sheet, increasing the flexibility. When the PSA sheet is highly flexible, the PSA sheet is more likely to deform to conform to unevenness or steps on an adherend surface, thereby allowing its adhesive face to tightly adhere to the adherend surface. According to the layer C with cushioning properties, when the layer C side adhesive face is applied to, for instance, a hard adherend, because of the layer C presence, the hard adherend shape and the like are absorbed into the layer C, preventing or reducing the influence on the opposite side of the PSA sheet. Increased flexibility of the PSA sheet may also help keep the PSA sheet in place. When the PSA sheet is adhered to conform to a curved surface or an uneven adherend surface, or when the adherend with the PSA sheet deforms, etc., the PSA sheet can be effectively suppressed from peeling off (floating above) the adherend surface due to its own repulsive force.

The layer C may comprise both a filler (e.g., hollow particles) and bubbles such as those described above. A PSA sheet comprising such a layer C is preferable since it is likely to have well-balanced flexibility and cohesive strength.

The bubbles in the layer C may be independent bubbles, successive bubbles, or a mixture of these. From the standpoint of the cushioning effect, a layer C comprising many independent bubbles is more preferable. With respect to independent bubbles, the gaseous component included in the bubbles (gas component forming the bubbles; or "bubble-forming gas" hereinafter) is not particularly limited, and it can be various gaseous components such as insert gases including nitrogen, carbon dioxide, argon, etc., as well as various gaseous components such as air, etc. When the polymerization, etc., are carried out in a state containing the bubble-forming gas, it is preferable to use, as the bubble-forming gas, a gas species that does not inhibit the reaction(s). From such a standpoint and in respect of cost, etc., nitrogen can be preferably used as the bubble-forming gas.

In typical, the shapes of bubbles are more or less globular while they are not limited to such shapes. The average diameter of bubbles (average bubble diameter) is not particularly limited. It can be selected, for instance, from a range of 1 µm to 1000 µm, preferably 10 µm to 500 µm, or more preferably 30 µm to 300 µm. The average bubble diameter is usually suitably 50 % of the thickness of the layer C or smaller, or preferably 30 % or smaller (e.g., 10 % or smaller).

The average bubble diameter can be determined typically by scanning electron microscopy (SEM), preferably by measuring 10 or more bubbles for their diameters and arithmetically averaging the results. For this, with respect to non-globular bubbles, they are converted to globular bubbles having the same volumes to determine the average bubble diameter.

When the layer C has bubbles, the volume ratio of the bubbles (bubble content, porosity) in the layer C is not particularly limited. It can be suitably selected so as to obtain aiming cushioning effect and flexibility. Relative to the layer C's volume (referring to its apparent volume, which can be determined from the thickness and area of the layer C, it can be about 3 % to 70 % by volume, or it is usually suitably about 5 % to 50 % by volume, or preferably about 8 % to 40 % by volume.

In the art disclosed herein, the method for forming a layer C having bubbles (bubble-containing viscoelastic layer) is not particularly limited, and a known method can be suitably employed. For example, can be suitably employed (1) a method such that a viscoelastic layer-forming composition pre-mixed with a bubble-forming gas (preferably a composition that forms a viscoelastic material upon curing by active energy rays such as UV rays, etc.) is cured to form a bubble-containing viscoelastic layer, (2) a method such that a viscoelastic layer-forming composition containing a foaming agent is used and by forming bubbles from the foaming agent, a form a bubble-containing viscoelastic layer is formed, and like method. The foaming agent used is not particularly limited. A suitable one can be selected from known foaming agents. For instance, foaming agents such as thermally expandable microspheres and the like can be preferably used.

In forming a bubble-containing viscoelastic layer by the method (1), the method for preparing the viscoelastic layer-forming composition pre-mixed with a bubble-forming gas is not particularly limited. A known bubble-mixing method can be used. For instance, a known bubble-mixing device can be used in the method. Examples of a bubble-mixing device include a device comprising a stator having fine teeth on a disc having a through-hole at the center, and a rotor opposing the stator and having fine teeth on a disc similarly to the stator. In between the stator teeth and rotor teeth in such a bubble-mixing device, a viscoelastic layer-forming composition prior to bubble mixing (viscoelastic layer-forming composition precursor) is introduced, and while the rotor is allowed to rapidly spin, a gas component for forming bubbles (bubble-forming gas) is introduced into the viscoelastic layer-forming composition precursor through the through-hole. By this, a viscoelastic layer-forming composition can be obtained with bubbles finely dispersed and mixed therein.

A bubble-containing viscoelastic layer can be formed by, for example, applying such a composition mixed with a bubble-forming gas to a prescribed surface and allowing it to cure. As the curing method, a heating method, a method where an active energy ray (e.g., UV ray) is irradiated, etc., can be preferably employed. By curing a viscoelastic layer-forming composition mixed with a bubble-forming gas by means of heating or irradiating an active energy ray, etc., while in a state where bubbles are stably retained, a bubble-containing viscoelastic layer can be preferably formed.

From the standpoint of the mixing compatibility of a bubble-forming gas or the stability of bubbles, a surfactant may be added to the viscoelastic layer-forming composition. Examples of such a surfactant include ionic surfactants, hydrocarbon-based surfactants, silicone-based surfactants, fluorine-based surfactants and the like. Among these, fluorine-based surfactants are preferable. In particular, a fluorine-based surfactant having an oxyalkylene group (typically an oxyalkylene group having 2 to 3 carbon atoms) along with a fluorinated hydrocarbon group is preferable. Fluorine-based surfactants can be used singly as one species or in combination of two or more species. Examples of preferably usable commercial fluorine-based surfactants include trade name SURFLON S-393 available from AGC Seimi Chemical Co., Ltd.

The amount of surfactant used is not particularly limited. For instance, it can be about 0.01 part to 3 parts by weight by non-volatiles to 100 parts by weight of the base polymer (e.g., acrylic polymer) in the layer C.

As far as the effects by the present invention are not significantly impaired, the layer C may include as necessary known additives such as a plasticizer, softening agent, colorants (pigments, dyes, etc.), antioxidant, leveling agent, stabilizing agent, and preservative.

The method for forming a layer-C-containing PSA sheet is not particularly limited. For instance, in a preferable method, a layer-C-forming composition is applied onto a releasable surface (release face, e.g., a release liner surface) and allowed to cure (e.g., by UV) to form a layer C, and the resulting layer C is adhered (transferred) to the layer A surface. Alternatively, for instance, a layer-C-forming composition can be applied to a layer A surface and allowed to cure (e.g., by UV) to form a layer-C-containing PSA sheet.

In some embodiments, as the layer C, it is possible to use a PSA-layer-containing double-faced PSA sheet with or without substrate. The PSA layer is formed of one, two or more species selected among various PSAs such as an acrylic PSA, rubber-based PSA, silicone-based PSA, polyester-based PSA, urethane-based PSA, polyether-based PSA, polyamide-based PSA, and fluorine-based PSA. In particular, a preferable layer C includes an acrylic PSA layer. For the details of the PSA, reference is made to the description of the viscoelastic materials for the layer C, so redundant description is not repeated here. The substrate of the double-faced PSA sheet with substrate is not particularly limited. It is possible to use resin film, paper, cloth, woven fabric, non-woven fabric, foam sheet, a composite of these, etc. In particular, nonwoven fabrics are preferable. One favorable example is a double-faced PSA sheet with substrate having an acrylic PSA layer on each face of a nonwoven fabric substrate.

The layer C thickness is not particularly limited and can be, for instance, 200 µm or greater. Being viscoelastic, the layer C is highly flexible. By arranging the layer C, the PSA sheet surface (adhesive face) can preferably provide tight adhesion to an adherend. From the standpoint of the flexibility, the layer C has a thickness of preferably 250 µm or larger, or more preferably 300 µm or larger (e.g., 350 µm or larger). From the standpoint of obtaining higher flexibility, the layer C thickness can be 500 µm or larger, or even 700 µm or larger. The art disclosed herein can be preferably implemented in an embodiment where the layer C has a thickness of 1 mm or larger. With increasing layer C thickness, the layer C tends to preferably show its effect (e.g., peel resistance on flexible and moving adherends such as human skin, properties of absorbing adherend structure, cushioning properties, etc.). The maximum layer C thickness is not particularly limited. For instance, it can be about 10 mm or smaller. From the standpoint of the ease of formation or cohesiveness of the layer C, etc., the layer C thickness is typically suitably 5 mm or smaller, or preferably 3 mm or smaller (e.g., 2 mm or smaller). In other embodiments, the layer C (e.g., possibly a double-faced PSA sheet with or without substrate) can have a thickness of about 30 µm or greater, about 50 µm or greater, about 80 µm or greater, or even 120 µm or greater. In such an embodiment, the layer C thickness can be, for instance, about 1 mm or less, about 500 µm or less, or even less than 300 µm (e.g., less than 200 µm). According to the art disclosed herein, with the PSA sheet having the layer A and the layer C with such a thickness, stable adhesive fixation can be obtained with respect to adherends adhered to both faces of the PSA sheet.

### <Other layers>

In addition to the layer A, the PSA sheet disclosed herein may optionally have other layers different from the aforementioned layers A, B, and C. Examples of the other layers include a layer placed between layers A and B, a layer placed between layers A and C, and a layer placed on the layer A's backside (the opposite side of the adhesive face, e.g., the opposite side of the layer B side of the layer A in an embodiment having a laminated structure of layer A and layer B (PSA layer)). Such other layers may support, for instance, layers A and B, or a layer C. These other layers are not particularly limited. Examples of such other layers include, but are not limited to, nonwoven fabrics, woven fabrics, and other porous films. As the nonwoven/woven fabrics and other porous films, it is possible to use, for instance, the materials described for the substrate layer of layer A. Redundant description is omitted here.

### <PSA sheet properties, etc.>

### (180° peel strength)

With respect to the adhesive face (the layer B-side adhesive face in an embodiment having a layer B) of the PSA sheet disclosed herein, the adhesive strength (180° peel strength on stainless steel plate) is not limited to a specific range, as it may vary depending on the purpose and application area. The PSA sheet has an adhesive strength of, for instance, possibly about 1 N/10mm or greater, or suitably about 2 N/10mm or greater. In view of adhesive reliability, the adhesive strength is preferably 4 N/10mm or greater, more preferably 4.5 N/10mm or greater, yet more preferably 5.0 N/1 0mm or greater, or possibly even 5.5 N/10mm or greater (e.g., 6.0 N/10mm or greater). The PSA sheet having such an adhesive strength can provide good adhesive fixation. For instance, it can show peel-resistant adhesion even to flexible and moving human skin. The maximum adhesive strength is not particularly limited. It can be about 12 N/10mm or less (e.g., 8 N/10mm or less). The adhesive strength can be determined by the method described later in Examples. In the case of a double-faced PSA sheet having adhesive faces on both sides, the two faces may be the same or different in adhesive strength.

### (Holding power)

The PSA sheet disclosed herein preferably has a displacement distance of less than 0.1 mm from the initial application position at 1 hour after the start of the test in a holding power test carried out by the method described later in Examples. Such a PSA sheet does not easily shift out of where it is applied, and can exhibit excellent adhesive reliability in long-term applications.

### (Double-faced PSA sheet)

The PSA sheet according to some preferable embodiments is a double-faced PSA sheet having layers B, A and C in this order. With such an embodiment, the layer B-side and layer C-side adhesive faces can be individually applied to different adherends, providing adhesive fixation. While no particular limitations are imposed, when the layer B-side adhesive face is applied to a flexible and moving adherend (typically human skin), the PSA sheet does not easily peel off from the adherend or shift out of place while peeling for removal can be achieved without damaging the adherend. When the layer C-side adhesive face is applied to, for instance, a hard adherend (e.g., a resin- or metal-containing sensor), the layer C presence can prevent or mitigate the shape, etc., of the hard adherend from affecting the layer B-side adherend. For instance, in the embodiment where the layer B side is applied to human skin, discomfort associated with the presence of the adherend attached to the layer C side can be eliminated or reduced.

### (Total thickness of PSA sheet)

The total thickness of the PSA sheet (including layers A to C, but not a release liner if any) disclosed herein is not particularly limited. In some preferable embodiments, the total PSA sheet thickness can be, for instance, about 12 mm or less. From the standpoint of the handling properties and formability, it is suitably about 5 mm or less, preferably about 2 mm or less, more preferably about 1.5 mm or less, or possibly even about 1 mm or less. The minimum thickness of the PSA sheet according to such embodiments is not particularly limited. For instance, it is suitably about 200 µm or greater, preferably about 400 µm or greater, more preferably about 600 µm or greater, or possibly even about 800 µm or greater. While no particular limitations are imposed, the total thickness can be preferably adopted as the total thickness of the PSA sheet having layers A, B and C. In other embodiments, the PSA sheet thickness is, for instance, suitably about 10 µm or greater, preferably about 50 µm or greater, more preferably about 80 µm or greater, or possibly even about 100 µm or greater. In this embodiment, the maximum PSA sheet thickness is not particularly limited and can be about 2500 µm or less. For instance, it can be about 1000 µm or less, 500 µm or less, 300 µm or less, or even 200 µm or less. The PSA sheet with a limited thickness is suitable for applications where reduction in weight and thickness is desirable.

### <Applications>

The purpose of the PSA sheet disclosed here is not particularly limited, and it can be used in applications. The PSA sheet disclosed herein has adhesive reliability with resistance to peeling and displacement as well as removability without damage to the adherend. Thus, it can be preferably used in applications where the adhesive reliability is required, but adherend damage is undesirable. For instance, it can be used for applications on adherends with brittle surfaces and adherends with low mechanical strength. In addition, it also has adhesive reliability that can endure long-term applications. Thus, it can also be used in applications requiring adhesive fixation for more than several days or weeks. In particular, the PSA sheet disclosed herein can be preferably used in an embodiment in which the adhesive surface (specifically, the layer B-side adhesive face) is directly or indirectly attached to the skin of a living body (typically a person). The PSA sheet applied on the skin can gently conform to the application area according to the body temperature, thereby bringing about properties to resist peeling and displacement. For instance, it can have resistance to peeling and displacement due to human movement. On the other hand, when the PSA sheet is removed, the PSA sheet suitably resists deformation during peeling and reduces the local concentration of stress on the adherend. Thus, removal of the PSA from the skin causes little tension (deformation) on the skin, allowing for gentle peeling with little or no pain.

The PSA sheet (typically a double-faced PSA sheet) disclosed herein is suitable for applications where hard adherends are attached to the skin. The type of hard adherend is not particularly limited. Various products and members formed of metal or resin and having prescribed shapes can be attached to human skin. When they are attached to the skin, their shape and hardness may be transmitted to the skin and cause discomfort. The PSA sheet disclosed herein may preferably have cushioning properties that absorb the adherend shape. Thus, when such an embodiment is employed, various products and the like can be fixed to the skin without discomfort.

Favorable examples of the hard adherend attached to the skin include various sensors. The art disclosed herein is particularly suitable for use in attaching a sensor that is attached to a living body such as a human and is capable of acquiring internal information and information about the movement of the living body. When a sensor or the like is fixed via the PSA sheet disclosed herein to a specific area of a person, the PSA sheet does not easily peel off or shift out of place. Thus, the sensing function associated with where the sensor is fixed can work with high accuracy. The use of the PSA sheet with the function of fixing sensors to parts of the human body allows medical personnel to obtain information such as the patient's health status without direct contact with the patient. For instance, it can be useful for consultation and treatment in home care and telemedicine. It can also be used in the fields of healthcare and preventive medicine. Furthermore, if the PSA sheet disclosed herein can be used to accurately detect the movement of each part of a person, it can also be expected to be used in the field of sports, for example. The PSA sheet disclosed herein can be useful in fields such as medicine, healthcare and sports science.

Based on the above, this description provides a PSA sheet with sensor attached, that is, a sensor-bearing PSA sheet. The sensor-bearing PSA sheet may have, for instance, a cross-sectional structure schematically illustrated in Fig. 4. In a sensor-bearing PSA sheet 300 shown in Fig. 4, a PSA sheet 2 is a double-faced PSA sheet in which a sensor 50 is fixed to its one adhesive face 2B. In this embodiment, the other adhesive face 2A of PSA sheet 2 is attached to human skin. Other features of PSA sheet 2 are basically the same as those shown in Fig. 3, so the description is not repeated.

It is noted that in an embodiment where the art disclosed herein is implemented in the form of an adhesively single-faced PSA sheet (single-faced PSA sheet), when using the single-faced PSA sheet for adherend fixation, one adherend can be fixed to the backside (non-adhesive face) of the single-faced PSA sheet using a known or commonly-used adhesive or PSA (possibly in the form of double-faced tape) and another adherend can be attached to the adhesive face of the single-faced PSA sheet. For instance, the PSA sheet disclosed herein can be preferably used in an embodiment where a sensor is fixed to the backside of a single-faced using double-faced PSA tape and the adhesive face of the single-faced PSA sheet is adhered to human skin.

### [Examples]

Several working examples related to the present invention are described below, but these specific examples are not to limit the present invention. In the description below, "parts" and "%" are by weight unless otherwise specified.

### <Experiment 1>

### [Preparation of resin sheet A]

### (Preparation Example Al)

To a flask equipped with a reflux condenser, thermometer and dropping funnel, were added 156 g of 2-ethylhexyl acrylate (2EHA), 36 g of N-vinyl-pyrrolidone (NVP), 8 g of 4-hydroxybutyl acrylate (4HBA), 0.2 g each of 2,2-dimethoxy-1,2-diphenylethane- I -one (available from IGM Resins, product name OMNIRAD 651) and 1-hydroxy-cyclohexyl phenyl ketone (available from IGM Resins, produce name OMNIRAD 184) as photopolymerization initiators; and the flask was purged with nitrogen. Subsequently, using a high-pressure mercury lamp (product name EXECURE 4000, available from HOYA CANDEO OPTRONICS CORPORATION), UV irradiation was carried out to obtain viscous liquid (UV syrup) containing a partial polymer of the monomer mixture. To 50 g of the UV symp, were added 50 g of isobomyl acrylate (IBXA) and 0.1 g of 1,6-hexanediol diacrylate (HDDA) as a crosslinking agent to obtain a UV-curable resin composition according to this Example.

To a 38 µm thick release film R1 (product name MRF #38 available from Mitsubishi Plastics, Inc.) formed of polyethylene terephthalate (PET) film with silicone release treatment on one face, the resulting resin composition was applied and then covered to block air with a 38 µm thick release film R2 (product name MRE #38 available from Mitsubishi Plastics, Inc.) formed of PET film with silicone release treatment (light-release treatment) on one face. The resultant was allowed to cure (complete polymerization) by UV irradiation (blacklight) to form a 100 µm thick resin sheet A1. The UV irradiation was performed using a blacklight lamp, at an intensity of 5 mW/cm² (measured with an industrial UV checker (trade name UVR-T1 available from Topcon Corporation) with peak sensitivity at ~350 nm wavelength) for 300 seconds. The respective faces of the resin sheet Al are protected with release films (release liners) R1 and R2.

### (Preparation Example A2)

In the same manner as Preparation Example A1, was prepared a UV-curable resin composition. To 50 g of the resulting resin composition, was added 50 g of water. The resultant was stirred at high speed for 2 minutes at 5000 rpm, using TK ROBOMIX (available from PRIMIX Corporation). In the same manner as Preparation Example A1, to a release film R1 (product name MRF #38 available from Mitsubishi Plastics, Inc.), the resulting resin composition emulsion was applied and then covered to block air with a release film R2 (product name MRE #38 available from Mitsubishi Plastics, Inc.). The resultant was allowed to cure (complete polymerization) by UV irradiation (blacklight) and then dried at 120 °C for 2 minutes in a dryer to form a 100 µm thick resin sheetA2. The resin sheet A2 is a porous resin sheet having an open-cell structure.

### (Preparation Example A3)

To 50 g of UV syrup prepared in Preparation Example A1, were added 75 g of IBXA and 0.1 g of HDDAto obtain a UV-curable resin composition according to this Example. Using the resulting resin composition, but otherwise in the same manner as Preparation Example A1, was formed a 100 µm thick resin sheet A3.

### (Preparation Example A4)

In the same manner as Preparation Example A3, was prepared a UV-curable resin composition. To 50 g of the resulting resin composition, was added 50 g of water. The resultant was stirred at high speed for 2 minutes at 5000 rpm, using TK ROBOMIX (available from PRIMIX Corporation). Using the resulting resin composition emulsion, but otherwise in the same manner as Preparation Example A2, was formed a 100 µm thick resin sheetA4. The resin sheet A4 is a porous resin sheet having an open-cell structure.

### (Preparation Example AS)

In the same manner as Preparation Example A1, was prepared a UV-curable resin composition. To 50 g of the resulting resin composition, was added 80 g of water. The resultant was stirred at high speed for 2 minutes at 5000 rpm, using TK ROBOMIX (available from PRIMIX Corporation). Using the resulting resin composition emulsion, but otherwise in the same manner as Preparation Example A2, was formed a 100 µm thick resin sheet A5. The resin sheet A5 is a porous resin sheet having an open-cell structure.

### (Preparation Example A6)

To a flask equipped with a reflux condenser, thermometer and dropping funnel, were added 72 g of 2EHA, 15 g of NVP, 3 g of AA and 100 g of IBXA as monomers; and were further added 1 g of product name KBM-503 ((y-methacryloxypropyl)trimethoxysilane available from Shin-Etsu Chemical Co., Ltd.) as a silane coupling agent, 10 g of an emulsifier (product name LATEMI, E-118B available from Kao Corporation), and 200 g of water. The resultant was emulsified for 5 minutes with a homomixer. The resulting emulsion was purged with nitrogen (for oxygen removal) for 1 hour and then heated to 60 °C. To this, was added 0.2 g of polymerization initiator (VA-057 available from Wako Pure Chemical Industries, Ltd.) and polymerization was carried out for 3 hours. Subsequently, the system was heated to 70 °C. Polymerization was further carried out for 3 hours and it was allowed to cool to room temperature. For every 100 g of the resulting polymer emulsion (water dispersion of polymer), was added 1 g of a thickener (product name A-30H available from Toagosei Co., Ltd.) to obtain a resin composition according to this Example. To a 38 µm thick release film R1 (product name MRF #38 available from Mitsubishi Plastics, Inc.) formed of PET film with silicone release treatment on one face, the resulting resin composition was applied and allowed to dry at 120 °C for 2 minutes to form a 100 µm thick resin sheet A6. The exposed face of the resulting resin sheetA6 was covered with a 38 µm thick release film R2 (product name MRE #38 available from Mitsubishi Plastics, Inc.) formed of PET film with silicone release treatment (light-release treatment) on one face to protect the two faces of the resin sheetA6 with release films (release liners) R1 and R2.

### (Preparation Example A7)

In the same manner as Preparation Example A6, was prepared a polymer emulsion (water dispersion of polymer). For every 100 g of the polymer emulsion, were added 1 g of a thickener (product name A-30H available from Toagosei Co., Ltd.), 2 g of a surfactant (product name AMOGEN CB-H available from DKS Co., Ltd.; carboxybetaine amphoteric surfactant), 0.5 g of a foam regulator (product name NOPCO DC-100-A available from San Nopco, Ltd.). The resultant was stirred at high speed for 5 minutes at 5000 rpm using TK ROBOMIX (available from PRIMIX Corporation). In the same manner as Preparation Example A6, to a release film R1 (product name MRF #38 available from Mitsubishi Plastics, Inc.), the resulting resin composition was applied and allowed to dry to form a 100 µm thick resin sheetA7. The exposed face of the resulting resin sheet A7 was covered and protected with a release film R2 (product name MRE #38 available from Mitsubishi Plastics, Inc.). The resin sheet A7 is a porous resin sheet having an open-cell structure.

### (Preparation Example A8)

The monomers were changed to 42 g of n-butyl acrylate (BA), 50 g of methyl methacrylate (MMA), 1 g of hydroxyethyl acrylate (HEA) and 7 g of polyethylene glycol monoacrylate (product name BLEMMERAE200 available from NOF Corporation). Otherwise, in the same manner under the same conditions as Preparation Example A6, polymerization was carried out to obtain a resin composition. In the same manner as Preparation Example A6, using the resulting resin composition, was obtained a 100 µm thick resin sheet A8 with the respective faces protected with release films R1 and R2 (product names MRF #38 and MRE #38 available from Mitsubishi Plastics, Inc.).

### (Preparation Example A9)

To 100 g of a commercial acrylic latex (product name AE986B available from Emulsion Technology Co., Ltd.), was added 1 g of a thickener (product name A-30H available from Toagosei Co., Ltd.). The resultant was stirred, mixed and then defoamed to obtain a resin composition according to this Example. Using the resulting resin composition, in the same manner as Preparation Example A6, was obtained a 100 µm thick resin sheet A9 with the respective faces protected with release films R1 and R2 (product names MRF #38 and MRE #38 available from Mitsubishi Plastics, Inc.).

### (Preparation Example A10)

In the same manner as Preparation Example A9, were mixed a commercial acrylic latex and a thickener. To 50 g of the mixture, were added 1 g of a surfactant (product name AMOGEN CB-H available from DKS Co., Ltd.; carboxybetaine amphoteric surfactant) and 0.25 g of a foam regulator (product name NOPCO DC-100-A available from San Nopco, Ltd.). The resultant was stirred at high speed for 5 minutes at 5000 rpm using TK ROBOMIX (available from PRIMIX Corporation). In the same manner as Preparation Example A6, was obtained a 100 µm thick resin sheet A10 with the respective faces protected with release films R1 and R2 (product names MRF #38 and MRE #38 available from Mitsubishi Plastics, Inc.). The resin sheet A10 is a porous resin sheet having an open-cell structure.

### (Preparation Example A11)

To 100 g of a commercial acrylic latex (product name NIPOL LX855EX1 available from Zeon Corporation), was added 1 g of a thickener (product name A-30H available from Toagosei Co., Ltd.). The resultant was stirred, mixed and then defoamed to obtain a resin composition according to this Example. Using the resulting resin composition, in the same manner as Preparation Example A6, was obtained a 100 µm thick resin sheet A11 with the respective faces protected with release films R1 and R2 (product names MRF #38 and MRE #38 available from Mitsubishi Plastics, Inc.).

### (Preparation Example A12)

To 100 g of a commercial urethane-based latex (product name SUPERFLEX 150HS available from DSK Co., Ltd.), was added 0.8 g of athickener (product name A-30H available from Toagosei Co., Ltd.). The resultant was stirred, mixed and then defoamed to obtain a resin composition according to this Example. Using the resulting resin composition, in the same manner as Preparation Example A6, was obtained a 100 µm thick resin sheet A12 with the respective faces protected with release films R1 and R2 (product names MRF #38 and MRE #38 available from Mitsubishi Plastics, Inc.).

### (Preparation Example A13)

In the same manner as Preparation Example A12, were mixed a commercial urethane-based latex and a thickener. To 50 g of the mixture, were added 1 g of a surfactant (product name AMOGEN CB-H available from DKS Co., Ltd.; carboxybetaine amphoteric surfactant) and 0.25 g of a foam regulator (product name NOPCO DC-100-A available from San Nopco, Ltd.). The resultant was stirred at high speed for 5 minutes at 5000 rpm using TK ROBOMIX (available from PRIMIX Corporation). In the same manner as Preparation Example A6, was obtained a 100 µm thick resin sheet A13 with the respective faces protected with release films R1 and R2 (product names MRF #38 and MRE #38 available from Mitsubishi Plastics, Inc.). The resin sheet A13 is a porous resin sheet having an open-cell structure.

### (Preparation Example A14)

To 100 g of a commercial acrylic latex (product name N975(A)1 available from Emulsion Technology Co., Ltd.), was added 1 g of a thickener (product name A-30H available from Toagosei Co., Ltd.). The resultant was stirred, mixed and then defoamed to obtain a resin composition according to this Example. Using the resulting resin composition, in the same manner as Preparation Example A6, was obtained a 100 µm thick resin sheet A14 with the respective faces protected with release films R1 and R2 (product names MRF #38 and MRE #38 available from Mitsubishi Plastics, Inc.).

### [Preparation of PSA layer B]

### (Preparation Example B1)

To a flask equipped with a reflux condenser, thermometer and dropping funnel, were added 190 g of 2EHA and 10 g of AA as monomers as well as 300 g of ethyl acetate as a solvent. After purged with nitrogen, the resultant was heated to 60 °C. To this, was added 0.2 g of 2,2'-azobisisobutyronitrile (AIBN) to initiate polymerization. After 3 hours of polymerization, the temperature was raised to 70 °C. Polymerization reaction was continued for 3 more hours to obtain a polymer solution. To 50 g of the polymer emulsion solution, was added 0.05 g of an isocyanate crosslinking agent (product name CORONATE HL available from Tosoh Corporation) and 50 g of ethyl acetate to prepare a PSA composition B1 according to this Example. To a 38 µm thick release film (product name MRF #38 available from Mitsubishi Plastics, Inc.) formed of PET film with silicone release treatment on one face, the resulting PSA composition was applied and allowed to dry at 120 °C for 2 minutes to form a 2 µm thick PSA layer B1.

### (Preparation Example B2)

The monomers were changed to 144 g of 2EHA, 50 g ofNVP and 6 g of AA. The amount of AIBN was changed to 0.4 g. Otherwise in the same manner as Preparation Example B1, was obtained a polymer solution. To 50 g of the polymer solution, were added 0.05 g of an isocyanate crosslinking agent (product name CORONATE HL available from Tosoh Corporation) and 50 g of ethyl acetate to prepare a PSA composition B2 according to this Example. Using the resulting PSA composition B2, in the same manner as Preparation Example B1, was obtained a 2 µm thick PSA layer B2.

### [Preparation of PSA layer C]

### (Preparation Example C1)

To a monomer mixture formed of 90 parts of 2EHA and 10 parts of AA, were added 0.05 part of product name OMNIRAD 651 (available from IGM Resins) and 0.05 part of product name OMNIRAD 184 (available from IGM Resins) as photopolymerization initiators. Subsequently, the resultant was irradiated by UV to a viscosity of about 15 Pa·s to prepare monomer syrup (partial polymer) resulting from partial polymerization of the monomer mixture. The viscosity was measured using a BH viscometer with a No. 5 rotor at a rotational speed of 10 rpm at a measurement temperature of 30 °C. To 100 parts of the monomer syrup, were added 0.10 part of dipentaerythritol hexaacrylate and 12.5 parts of hollow glass balloons (40 µm average particle diameter, trade name FUJI BALLOON H-40 available from Fuji Silysia Chemical Ltd.). The resultant was defoamed. After defoamed, was added 0.7 part of a fluorine-based surfactant (trade name SURFLON S-393 available from AGC Seimi Chemical Co., Ltd.) to obtain a PSA composition precursor. Using a bubble-mixing device, the PSA composition was stirred with nitrogen gas introduced through the through-hole of the device to obtain a PSA composition C1 with bubbles dispersed and mixed.

Were obtained two sheets of 38 µm thick polyethylene terephthalate (PET) films having a release face treated with a silicone-based release agent on one side. To 100 parts of the PSA composition, was added 0.04 part of product name OMNIRAD 651 (available from IGM Resins). The resultant was applied to the release face of the first sheet of PET film and covered with the release face of the second sheet of PET film. Both surfaces were cured by UV irradiation at an intensity of 5 mW/cm² for 3 minutes. For the UV irradiation, trade name BLACK LIGHT available from Toshiba Corporation was used. UV rays were measured using an industrial UV checker (product name UVR-T1 available from Topcon Corporation) with about 350 nm peak sensitivity wavelength. An 800 µm thick PSA layer C1 was thus formed. The PSA layer C1 is covered with release liner (PET film) on each side. The PSA layer C1 had about 20 % bubbles by volume.

### [Preparation of PSA sheet]

### (Example 1)

The release liner covering one face of the resin sheet (resin layer) A1 was removed. To the exposed face of the resin sheet A1, was adhered the PSA layer B1. The resultant was autoclaved (50 °C, 5 atm, 15 minutes) to obtain a laminate formed of the resin layer A1 as a layer A and the PSA layer B1 as a layer B. Subsequently, were removed the release liner covering the other face of the resin layer A1 and the release liner covering one face of the PSA layer C1. To the exposed face of the resin layer A1 in the laminate, was adhered the exposed face of the PSA layer C1 to obtain a double-faced PSA sheet with the PSA layer B1, the resin layer A1 and the PSA layer C1 as layers B, A and C laminated in this order.

### (Examples 2-14 and Comparative Examples 1-4)

Changes were made to combine resin layers A1 to A14 as layers A, PSA layers B1 to B2 as layers Band PSA layers C1 as layers C as shown in Table 1. Otherwise in the same manner as Example 1, were obtained double-faced PSA sheets according to the respective Examples.

### <Evaluations>

### [Nanoindentation]

With respect to the resin sheet A (i.e., the resin layer A or layer A) according to each Example, nanoindentation was carried out to determine surface hardness (MPa) at 37 °C, unloading curve displacement (nm) and area (pJ) inside a load-displacement curve(s) at load ≤ 0. In particular, the release liner protecting the PSA sheet A surface was removed. A1 cm × 1 cm sized piece was cut out and set on a measurement table. Using a nanoindenter (TRIBOINDENTER available from Hysitron) under the conditions shown below, measurements were carried out to determine surface hardness (MPa) at 37 °C, unloading curve displacement (nm) and area (pJ) inside a load-displacement curve at load ≤ 0.

### (Measurement conditions)

Indenter used: Berkovich (trigonal pyramid) diamond indenter
Measurement method: Single indentation measurement
Measurement temperature: 37 °C atmosphere
Indentation depth: 1 µm (1000 nm)
Indentation rate: 1000 nm/s
Removal rate: 1000 nm/s

### [Storage modulus and Tg]

The resin sheet A alone was pasted together to a thickness of about 1 mm. This was punched out to ϕ 8 mm to prepare a columnar pellet as a measurement sample. The measurement sample was fixed to a ϕ 8 mm parallel plate jig. Using a dynamic viscoelastic analyzer (ARES G2 available from TA Instruments, Inc.), measurements were made under the following conditions to determine storage moduli G' (MPa) at 25 °C and 37 °C as well as loss tangent tan δ (G"/G') peak temperature as the glass transition temperature Tg (°C).

### (Conditions)

Measurement mode: shear mode
Temperature range: -50 °C to 150 °C
Heating rate: 5 °C/min
Frequency: 1 Hz

It is noted that with respect to the PSA layers B, measurements of 25°C storage modulus G' and Tg were performed by the same methods as the above; the PSA layer B1 was determined to have a 25°C storage modulus of 0.6 MPa and a Tg of °C; and the PSA layer B2 was determined to have a 25°C storage modulus of 2.2 MPa and a Tg of 11 °C.

### [180° peel strength (adhesive strength)]

With respect to the PSA sheet according to each Example, the release liner covering the layer C (PSA layer C) side adhesive face was removed and a 25 µm thick PET film (product name LUMIRROR S10 available from Toray Industries, Inc.) was adhered as backing to obtain a measurement sample. The measurement sample was cut into 5 cm × 1 cm size. The layer B (PSA layer B) side adhesive face was exposed and adhered to a stainless steel plate (SUS304BA plate) as an adherend. After the resultant was autoclaved, using a tensile testing machine (product name AUTOGRAPHAG-Xplus, HS 6000 mm/min high-speed model (AG-50NX plus) available from Shimadzu Corporation), while peeling off the PSA sheet from the adherend at a peel angle of 180° at a peel speed (tensile speed) of 300 mm/min, the load was measured to determine peel strength (N/10mm).

### [Holding power]

Based on JIS Z0237:2009, a holding power test was conducted. In particular, in an environment at 23 °C and 50 %RH, to the layer C (PSA layer C) side adhesive face of a double-faced PSA sheet, was adhered a 50 µm thick PET film as backing. The resultant was cut into a 20 mm wide strip to prepare a measurement sample. Subsequently, the layer B (PSA layer B) side adhesive face of the measurement sample was adhered to a Bakelite plate as an adherend with a 2 kg roller moved back and forth once. The bonding area between the measurement sample and the adherend was 20 mm wide and 20 mm long. In an environment at 40 °C, the measurement sample thus adhered to the adherend was vertically suspended and left for 30 minutes. A 500 g load was then applied to the free end of the measurement sample and with the load applied, the sample was left for 1 hour in the environment at 40 °C. With respect to the sample after left for 1 hour, the displacement distance (mm) from the initially-adhered position was measured. When the sample fell off the adherend within 1 hour, it was recorded as "Fall."

### [Peel stress distribution analysis]

The stress distribution during peeling was analyzed, and the peel resistance was evaluated from the stress integral value. The PSA sheet (formed of three layers) according to each Example was punched into ϕ 5 mm to obtain a measurement sample. The measurement sample was fixed onto an evaluation glass plate and pushed in until a stainless steel probe (5 mm in diameter) detected 100 N. Then, after maintained for 30 seconds, while peeling at a tensile speed (probe-raising speed) of 10 mm/min, the behavior was observed with a video camera, and the strain-stress distribution was measured. From the resulting values, were determined the integrated value of peel stress and the maximum value of peel stress at the beginning of peeling. With increasing peel stress integral value, the amount of energy required for peeling increases, making it more difficult to peel off. It is thought that with increasing maximum peel stress, the adherend damage caused by local stress concentration increases.

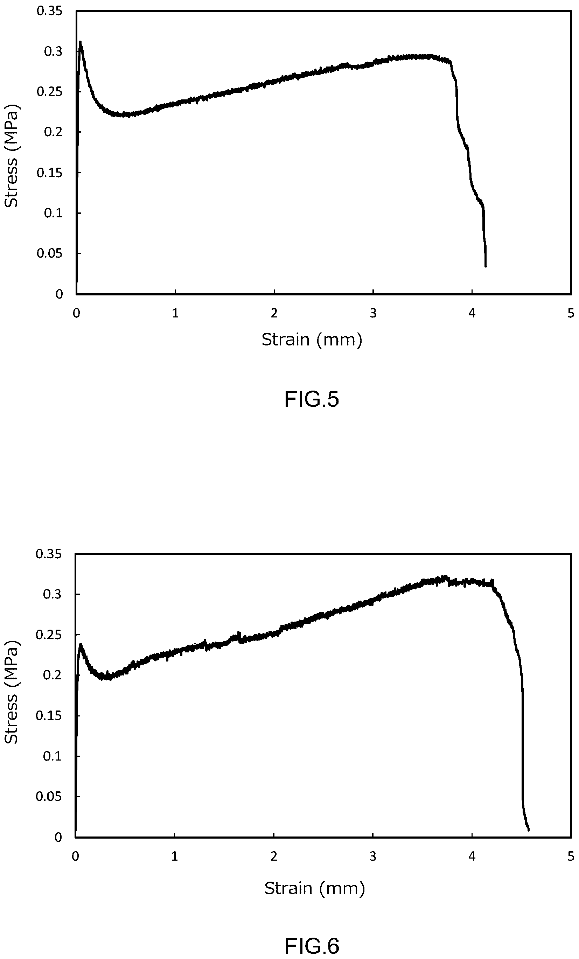

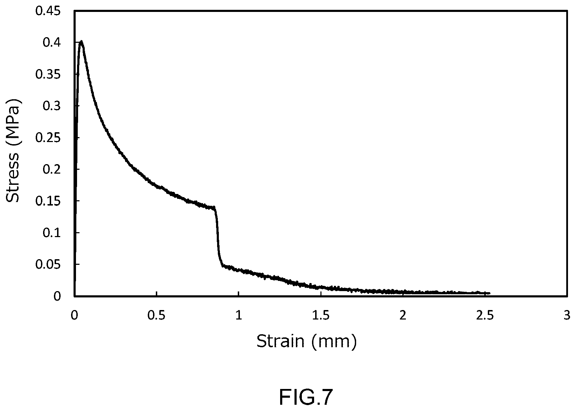

With respect to each Example, Table 1 shows the measurement results of resin sheet A (layer A) including surface hardness (MPa), 25°C storage modulus (MPa), 37°C storage modulus (MPa), Tg (°C), unloading curve displacement (nm), area (pJ) inside a load-displacement curve at load ≤ 0, layer structure of PSA sheet, adhesive strength (N/10mm), holding power (mm), peel stress integral value and maximum peel stress value. Figs. 5 to 7 show the peel stress distribution analysis results (stress-strain curves) of the PSA sheets according to Examples 1, 2 and Comparative Example 1, respectively.

### [Table 1]

**Table 1**

| | Resin sheet A (layer A) | | | | | | | PSA sheet | | | | |
|---|---|---|---|---|---|---|---|---|---|---|---|---|
| | Species | Surface hardness (MPa) | Unloading curve displacement (nm) | Area inside load displacement curve (pJ) | 25°C storage modulus (MPa) | 37°C storage modulus (MPa) | Tg (°C) | Layer structure (layers B/A/C) | Adhesive strength (N/10mm) | Holding power (mm) | Peel stress integral value | Maximum peel stress (MPa) |
| Ex. 1 | A1 | 0.4 | 1065 | 12.4 | 105 | 1.1 | 30 | B1/A1/C1 | 5.4 | 0 | 1.41 | 0.31 |
| Ex. 2 | A2 | 0.3 | 1202 | 14.1 | 79 | 0.8 | 30 | B1/A2/C1 | 4.3 | 0 | 1.18 | 0.24 |
| Ex. 3 | A3 | 1.1 | 410 | 19.2 | 471 | 16 | 38 | B1/A3/C1 | 4.7 | 0 | 1.25 | 0.31 |
| Ex. 4 | A4 | 0.7 | 452 | 24.9 | 202 | 11 | 38 | B1/A4/C1 | 4.2 | 0 | 1.08 | 0.26 |
| Ex. 5 | A5 | 0.3 | 1417 | 20.6 | 47 | 0.6 | 28 | B1/A5/C1 | 4.6 | 0 | 0.85 | 0.24 |
| Ex. 6 | A6 | 0.3 | 697 | 11.5 | 62 | 0.7 | 27 | B1/A6/C1 | 4.9 | 0 | 1.16 | 0.33 |
| Ex. 7 | A7 | 0.2 | 591 | 10.1 | 11 | 0.5 | 27 | B1/A7/C1 | 4.5 | 0 | 1.05 | 0.31 |
| Ex. 8 | A8 | 0.4 | 763 | 14.3 | 35 | 0.8 | 24 | B1/A8/C1 | 4.2 | 0 | 1.11 | 0.34 |
| Ex. 9 | A9 | 0.4 | 520 | 9.0 | 73 | 1.0 | 22 | B1/A9/C1 | 4.4 | 0 | 1.26 | 0.31 |
| Ex. 10 | A10 | 0.3 | 781 | 11.5 | 55 | 0.7 | 22 | B1/A10/C1 | 4.3 | 0 | 1.01 | 0.29 |
| Ex. 11 | A1 | 0.4 | 1065 | 12.4 | 105 | 1.1 | 30 | B2/A1/C1 | 6.2 | 0 | 1.43 | 0.34 |
| Ex. 12 | A2 | 0.3 | 1202 | 14.1 | 79 | 0.8 | 30 | B2/A2/C1 | 5.3 | 0 | 1.22 | 0.30 |
| Ex. 13 | A9 | 0.4 | 520 | 9.0 | 73 | 1.0 | 22 | B2/A9/C1 | 5.8 | 0 | 1.38 | 0.36 |
| Ex. 14 | A10 | 0.3 | 781 | 11.5 | 55 | 0.7 | 22 | B2/A10/C1 | 5.1 | 0 | 1.35 | 0.32 |
| Cmp. Ex. 1 | A11 | 320 | 0 | 1.1 | 15 | 24 | 36 | B1/A11/C1 | 1.1 | 0 | 0.25 | 0.41 |
| Cmp. Ex. 2 | A12 | 7.2 | 0 | 2.0 | 470 | 236 | 45 | B1/A12/C1 | 0.8 | 0.5 | 0.31 | 0.40 |
| Cmp. Ex. 3 | A13 | 6.7 | 0 | 2.2 | 160 | 4.8 | 36 | B1/A13/C1 | 0.9 | 0.2 | 0.21 | 0.42 |
| Cmp. Ex. 4 | A14 | 0.05 | - | - | 3.4 | 2.6 | -28 | B1/A14/C1 | 2.5 | Fall | 0.14 | 0.71 |

As shown in Table 1, with respect to the PSA sheets according to Examples 1 to 14 laminated with resins (A1 to A10) having 25°C storage moduli in the 10 MPa to 500 MPa range, 37°C storage moduli in the 0.5 MPa to 20 MPa range, and 37°C surface hardness in the 0.1 MPa to 2 MPa range, while having relatively high adhesive strength and holding power with large peel stress integral values, their maximum peel stress values were suppressed to less than 0.4 MPa. On the other hand, the PSA sheets according to Comparative Examples 1 to 4 not satisfying at least one among the 25°C storage modulus of 10 MPa to 500 MPa, the 37°C storage modulus of 0.5 MPa to 20 MPa, and the 37°C surface hardness of 0.1 MPa to 2 MPa, when compared with Examples 1 to 14, the adhesive strength was lower with a tendency towards inferior holding power; and the peel stress integral values were lower while the maximum peel stress values were 0.4 MPa or greater. Among Comparative Examples 1 to 4, none satisfied the adhesive strength of 4 N/10mm or greater.

These results indicate that a PSA sheet that is resistant to peeling/displacement and can be peeled off for removal without adherend damage can be obtained by using a resin that has a storage modulus at 25 °C in the range of 10 MPa to 500 MPa, a storage modulus at 37 °C in the range of 0.5 MPa to 20 MPa, and a surface hardness at 37 °C in the range of 0.1 MPa to 2 MPa.

### <Experiment 2>

### [Preparation of resin sheet A]

### (Preparation Example A2-1)

To the release face of a release film R1 (product name MRF #38 available from Mitsubishi Plastics, Inc.), was applied a UV-curable resin composition obtained by the same method as Preparation Example A1 in Experiment 1 to a cured thickness of 30 µm. Subsequently, a wet polyester nonwoven fabric substrate (product name SONTARA #8010 available from Jacob Holm) was adhered and covered on top to block air with a release film R2 (product name MRE #38 available from Mitsubishi Plastics, Inc.). The resultant was allowed to cure (complete polymerization) by UV irradiation (blacklight) to form a resin sheet A2-1. The UV irradiation was performed using a blacklight lamp, at an intensity of 5 mW/cm² (measured with an industrial UV checker (trade name UVR-T1 available from Topcon Corporation) with peak sensitivity at ~350 nm wavelength) for 300 seconds. The respective faces of the resin sheet A2-1 with nonwoven fabric substrate are protected with release films (release liners) R1 and R2.

### (Preparation Example A2-2)

To the release face of a release film R1 (product name MRF #38 available from Mitsubishi Plastics, Inc.), was applied a resin composition obtained by the same method as Preparation Example A9 in Experiment 1 to a cured thickness of 30 µm. Subsequently, a wet polyester nonwoven fabric substrate (product name SONTARA #8010 available from Jacob Holm) was adhered. The resultant was dried at 110 °C for 3 minutes to obtain a resin sheetA2-2 with nonwoven fabric substrate.

### [Preparation of PSA layer B]

### (Preparation Example B2-1)

To the release face of a release film R1 (product name MRF #38 available from Mitsubishi Plastics, Inc.), was applied a PSA composition B1 obtained by the same method as Preparation Example B1 in Experiment 1 and allowed to dry at 120 °C for 2 minutes to obtain a 30 µm thick PSA layer B2-1.

### (Preparation Example B2-2)

A 60 µm thick PSA layer B2-2 was obtained in the same manner as Preparation Example B2-1 except that the thickness was changed.

### (Preparation Example B2-3)

Were added 130 g of isononyl acrylate, 60 g of 2-methoxyethyl acrylate and 10 g ofAA as monomers as well as 75 g of toluene as a solvent. The resultant was purged with nitrogen and then heated to 60 °C. 0.4 g ofAIBN was added and reaction was carried out for 3 hours while maintaining the temperature at 60 °C. During the reaction, 140 g of toluene was added dropwise while observing the viscosity. After this, it was heated to 70 °C and polymerization reaction was carried out for 3 more hours to obtain a polymer solution. To 50 g of the polymer solution, was added 0.05 g of an isocyanate crosslinking agent (product name CORONATE HL available from Tosoh Corporation) and 50 g of toluene to prepare a PSA composition B2-3 according to this Example. Using the resulting PSA composition B2-3, in the same manner as Preparation Example B2-1, was obtained a 30 µm thick PSA layer B2-3.

### (Preparation Example B2-4)

A 60 µm thick PSA layer B2-4 was obtained in the same manner as Preparation Example B2-3 except that the thickness was changed.

### [Preparation of PSA sheet]

### (Example 15)

From a resin sheet (resin layer) A2-1, was removed the release liner R1. To the exposed face of the resin sheet A2-1, was adhered a PSA layer B2-1 to obtain a single-faced PSA sheet as a laminate of the resin layer A2-1 with nonwoven fabric substrate as a layer A and the PSA layer B2-1 as a layer B.

### (Example 16)

From a resin sheet (resin layer) A2-1, was removed the release liner R1. To the exposed face of the resin sheet A2-1, was adhered a PSA layer B2-3 to obtain a single-faced PSA sheet as a laminate of the resin layer A2-1 with nonwoven fabric substrate as a layer A and the PSA layer B2-3 as a layer B.

### (Example 17)

From a resin sheet (resin layer) A2-2, was removed the release liner R1. To the exposed resin layer surface, was adhered a PSA layer B2-1 to obtain a single-faced PSA sheet as a laminate of the resin layer A2-2 with nonwoven fabric substrate as a layer A and the PSA layer B2-1 as a layer B.

### (Example 18)

From a resin sheet (resin layer) A2-2, was removed the release liner R1. To the exposed resin layer surface, was adhered a PSA layer B2-3 to obtain a single-faced PSA sheet as a laminate of the resin layer A2-2 with nonwoven fabric substrate as a layer A and the PSA layer B2-3 as a layer B.

### (Comparative Example 5)

To a polyester nonwoven fabric substrate (product name SONTARA #8010 available from Jacob Holm), was adhered a PSA layer B2-2 to obtain a single-faced PSA sheet with nonwoven fabric substrate according to this Example.

### (Comparative Example 6)

To a polyester nonwoven fabric substrate (product name SONTARA #8010 available from Jacob Holm), was adhered a PSA layer B2-4 to obtain a single-faced PSA sheet with nonwoven fabric substrate according to this Example.

### <Evaluations>

### [Measurement of repulsion resistance on wrist]

To a 10 cm long and 1 cm wide cut piece of 1 mm thick polyolefin film, was adhered one adhesive face of commercial double-faced tape (No. 5000NS available from Nitto Denko Corporation). To the other adhesive face of the double-faced tape, was adhered the nonwoven fabric side surface of the single-faced PSA sheet according to each Example (Examples 15-18 and Comparative Examples 5-6). Subsequently, was removed the release liner covering the adhesive face of the single-faced PSA sheet. The exposed adhesive face was attached to a person's wrist. After left for 5 minutes, the height (cm) of the edge lifting (floating) from the wrist was measured to evaluate repulsion resistance.

### [120 g holding power test using gel with human skin texture]

To the backside (nonwoven fabric substrate side surface) of the single-faced PSA sheet according to each Example (Examples 15-18 and Comparative Examples 5-6), was adhered one adhesive face of commercial double-faced tape (No.5000NS available from Nitto Denko Corporation). The resultant was cut into a 1 cm square to obtain a measurement sample. The other adhesive face of the double-faced tape of the measurement sample was attached to a metal plate (total weight: 120 g) with a weight fixed to it, and the adhesive face of the single-faced PSA sheet on the opposite side was adhered to the surface of 1 mm thick human skin-like gel (hitohada gel) (product name H0-1K available from Exseal Co., Ltd.) fixed to a SUS plate. The time (min) from the adhesion until the weight dropped was measured.

### [Long-term skin adhesion test]

To a 10 cm long and 1 cm wide cut piece of 250 µm thick polyimide film, was adhered one adhesive face of commercial double-faced tape (No. 5000NS available from Nitto Denko Corporation). To the other adhesive face of the double-faced tape, was adhered the nonwoven fabric side surface of the single-faced PSA sheet according to each Example (Examples 15-18 and Comparative Examples 5-6). Subsequently, was removed the release liner covering the adhesive face of the single-faced PSA sheet. The exposed adhesive face was attached to a person's outer ann. The number of days or the time until an edge of the single-faced PSA sheet lifted from the outer arm (application period) and the sensation during application (wear feel) were evaluated. When no lifting was observed, the application was continued for a maximum of two weeks. As for the sensation during application, results were divided into cases with and without discomfort during application, and with respect to the cases with discomfort, the symptoms were recorded.

Table 2 shows the test results of Examples 15 to 18 and Comparative Examples 5 to 6. The resin sheets and PSA sheets according to the respective examples were evaluated in the same manner as Experiment 1. With respect to Examples 15 to 18, adhesive strength (N/10mm) and holding power (mm) were measured by the same methods as Experiment 1 except that PET film backing was omitted. In Experiment 2, the peel stress integral value and the maximum peel stress were determined in the same manner as in Experiment 1 except that the measurement sample was obtained by adhering commercial double-sided tape (No. 5000NS available from Nitto Denko Corporation) to the backside of the single-faced PSA sheet according to each Example. Table 2 also summarizes the features of each Example.

### [Table 2]

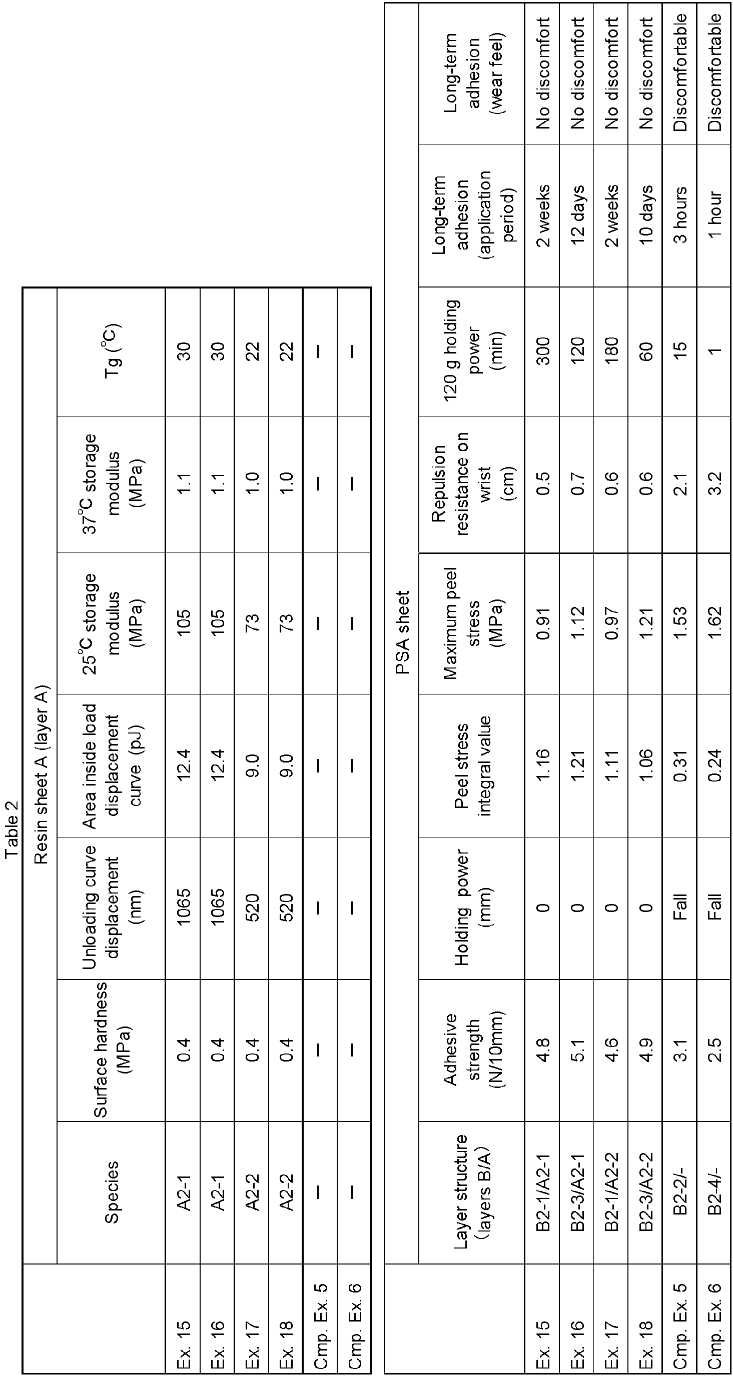

As shown in Table 2, in the repulsion resistance test on wrist, the PSA sheets according to Examples 15 to 18 with the layer A showed less than 1 cm of edge lifting while Comparative Examples 5 and 6 without layer A showed a large lifting height of 2 cm or more. In the holding power test with 120 g weight on human skin-like gel, in Examples 15 to 18 with the layer A, the shortest time to drop was 60 minutes, and the longest fixation time was 300 minutes without dropping. However, in Comparative Examples 5 and 6 without the layer A, both dropped shortly. In the long-term adhesion test using 250 µm thick polyimide film, in Comparative Examples 5 and 6 without the layer A, from the beginning, there was a tight feeling (discomfort) associated with tightening of skin; and within a few hours, edge lifting was observed and peeling started from there. On the other hand, in Examples 15 to 18, no discomfort such as tightness of the skin was observed throughout the application period. The application could be continued for 10 days or more, indicating excellent long-term adhesion properties.

Although specific embodiments of the present invention have been described in detail above, these are merely for illustrations and do not limit the scope of claims. The art according to the claims includes various modifications and changes made to the specific embodiments illustrated above.

### [Reference Signs List]

1, 2 PSA sheets
10 resin sheet, layer A
20 layer B
30 layer C
41, 42 release liners
50 sensor

## Claims

1. A resin for use in a pressure-sensitive adhesive sheet, the resin having
a storage modulus at 25 °C in the range of 10 MPa to 500 MPa,
a storage modulus at 37 °C in the range of 0.5 MPa to 20 MPa, and
a surface hardness at 37 °C in the range of 0.1 MPa to 2 MPa.

2. The resin according to Claim 1, having an unloading curve displacement in the range of 400 nm to 1500 nm in nanoindentation carried out at a temperature of 37 °C, an indentation depth of 1000 nm, and indentation/removal rates of 1000 nm/s.

3. The resin according to Claim 1 or 2, having an area inside a load-displacement curve of 5 pJ or greater when the load is 0 or less in nanoindentation carried out at a temperature of 37 °C, an indentation depth of 1000 nm, and indentation/removal rates of 1000 nm/s.

4. The resin according to any one of Claims 1 to 3, having a glass transition temperature in the range of 5 °C to 40 °C.

5. The resin according to any one of Claims 1 to 4, wherein the resin is an acrylic resin.

6. A pressure-sensitive adhesive sheet having a layer A formed from the resin according to any one of Claims 1 to 5.

7. The pressure-sensitive adhesive sheet according to Claim 6, having an adhesive face that has a 180° peel strength on stainless steel plate of 4 N/10mm or greater.

8. A pressure-sensitive adhesive sheet having a layer A, wherein
the layer A has a storage modulus at 25 °C in the range of 10 MPa to 500 MPa and a storage modulus at 37 °C in the range of 0.5 MPa to 20 MPa, and
the pressure-sensitive adhesive sheet has an adhesive face that has a 180° peel strength on stainless steel plate of 4 N/10mm or greater.

9. The pressure-sensitive adhesive sheet according to any one of Claims 6 to 8, having a layer B constituting the adhesive face, in addition to the layer A.

10. The pressure-sensitive adhesive sheet according to Claim 9, wherein the layer B is an acrylic pressure-sensitive adhesive layer.

11. The pressure-sensitive adhesive sheet according to Claim 9 or 10, wherein the layer B has a thickness of 0.5 µm or greater and 100 µm or less.

12. The pressure-sensitive adhesive sheet according to any one of Claims 6 to 11, having a layer C in addition to the layer A, wherein the layer C is placed on an opposite side of the adhesive face of the layer A.

13. The pressure-sensitive adhesive sheet according to Claim 12, wherein the layer C comprises hollow particles, or has bubbles, or has both.

14. The pressure-sensitive adhesive sheet according to Claim 12 or 13, wherein the layer C is an acrylic pressure-sensitive adhesive layer.

15. The pressure-sensitive adhesive sheet according to any one of Claims 6 to 14, for use in adhering the adhesive face to human skin.

16. The pressure-sensitive adhesive sheet according to any one of Claims 6 to 15, for use in fixing a sensor to human skin.
